(19) Europäisches Patentamt
European Patent Office
Office européen des brevets

(11) EP 2 309 974 B1

(12) # EUROPEAN PATENT SPECIFICATION

(45) Date of publication and mention of the grant of the patent:
**31.08.2016 Bulletin 2016/35**

(21) Application number: **09777441.8**

(22) Date of filing: **27.07.2009**

(51) Int Cl.:
***A61Q 19/02*** (2006.01) ***A61K 8/49*** (2006.01)

(86) International application number:
**PCT/EP2009/005406**

(87) International publication number:
**WO 2010/017885 (18.02.2010 Gazette 2010/07)**

(54) **USE OF 5-(7-METHOXY-3,3-DIMETHYL-2,3-DIHYDRO-1-BENZOXEPIN-5-YL)-3-METHYL-PENTA-2,4-DIENOIC**

VERWENDUNG VON 5-(7-METHOXY-3,3-DIMETHYL-2,3-DIHYDRO-1-BENZOXEPIN-5-YL)-3-METHYL-PENTA-2,4-DIENSÄURE

UTILISATION DE 5-(7-METHOXY-3,3-DIMETHYL-2,3-DIHYDRO-1-BENZOXEPIN-5-YL)-3-METHYL-PENTA-2,4-DIENOÏQUE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **12.08.2008 EP 08014354**

(43) Date of publication of application:
**20.04.2011 Bulletin 2011/16**

(73) Proprietor: **Merck Patent GmbH
64293 Darmstadt (DE)**

(72) Inventors:
- **ANZALI, Soheila
64846 Gross-Zimmern (DE)**
- **CONTARD, Francis
F-69008 Lyon (FR)**
- **ZEILLER, Jean Jacques
F-69008 Lyon (FR)**

(56) References cited:
**EP-A1- 0 671 161** **JP-A- 11 035 460**
**US-A- 5 225 436** **US-B1- 6 596 758**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 309 974 B1

## Description

The present invention relates to the nonotherapeutic use of 5-(7-methoxy-3,3-dimethyl-2,3-dihydro-1-benzoxepin-5-yl)-3-methyl-penta-2,4-dienoic acid and/or a salt or solvate thereof for skin lightening and/or skin whitening.

5-(7-methoxy-3,3-dimethyl-2,3-dihydro-1-benzoxepin-5-yl)-3-methyl-penta-2,4-dienoic acid is peroxisome proliferator-activated receptors (PPAR) ligand, in particular PPAR-alpha and -gamma agonist and has the following chemical structure

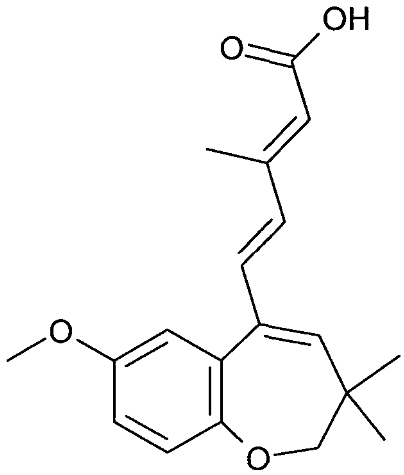

5-(7-methoxy-3,3-dimethyl-2,3-dihydro-1-benzoxepin-5-yl)-3-methyl-penta-2,4-dienoic acid is described as compound 16 in US 6,596,758 B1.

5-(7-methoxy-3,3-dimethyl-2,3-dihydro-1-benzoxepin-5-yl)-3-methyl-penta-2,4-dienoic acid is hereinafter also referred to as "Compound".

The subject of the patent refers to the definition of the claims, any disclosure that goes beyond the scope of the claims is for informational purposes only.

The human skin is subject to certain ageing processes, some of which are attributable to intrinsic processes (chronoageing) and some of which are attributable to exogenous factors (environmental, for example photoageing). In addition, temporary or even lasting changes to the skin picture can occur, such as acne, greasy or dry skin, keratoses, rosaceae, light-sensitive, inflammatory, erythematous, allergic or autoimmune-reactive reactions, such as dermatosis and photormatosis.

The exogenous factors include, in particular, sunlight or artificial radiation sources having a comparable spectrum, and compounds which can be formed by the radiation, such as undefined reactive photoproducts, which may also be free-radical or ionic. These factors also include cigarette smoke and the reactive compounds present therein, such as ozone, free radicals, for example the hydroxyl free radical, singlet oxygen and other reactive oxygen or nitrogen compounds which interfere with the natural physiology or morphology of the skin.

The influence of these factors can result, inter alia, in direct damage to the DNA of the skin cells and to the collagen, elastin or glycosaminoglycan molecules of the extracellular matrix, which are responsible for the strength of skin. In addition, the signal transduction chains, which are terminated by the activation of matrix-degrading enzymes, may be affected. Important representatives of these enzymes are the matrix metalloproteinases (MMPs, for example collagenases, gelatinases and stromelysins), whose activity is additionallly regulated by TIMPs (tissue inhibitors of matrix metalloproteinases).

The consequences of the above-mentioned ageing processes are thinning of the skin, weaker interlacing of epidermis and dermis, and a reduction in the number of cells and the supplying blood vessels. These results in the formation of fine lines and wrinkles, the skin becomes leathery, and pigment defects can occur.

The same factors also act on hair, where damage can likewise occur. The hairs become brittle, less elastic and dull. The surface structure of the hairs is damaged.

Cosmetic or dermatological care products having properties which are claimed to counter the processes described or comparable processes or reduce or reverse the harmful consequences thereof are frequently distinguished by the following specific properties - free-radical-scavenging, antioxidative, inflammation-inhibiting or humectant. They prevent or reduce, inter alia, the activity of matrix-degrading enzymes or regulate the new synthesis of collagen, elastin or proteoglycans.

The above-mentioned ageing processes result in a thinning of the skin, the decrease of serration between

epidermis and dermis, reduction of the cell number as well as the reduction of the supplying blood vessels. These processes are accompanied by the formation of lines and wrinkles, the skin becomes leather-like and/or shows pigmentary abnormalities.

Those factors also effect the status of the hair, resulting as well in a damage of the hair, especially damages in the surface of the hair that lead to brittleness and the loss of elasticity and gloss of the hair.

Care products and/or cosmetic products with properties that shall counteract against the described or similar processes and/or that shall reverse the damaging results often sure one or more of the following properties: free radical scavenging, anti-oxidative, anti-inflammatory and/or moisturising. Preferably, they block or reduce the activity of the matrix-disintegrating enzymes or control the de novo-synthesis of collagen, elastin and/or proteoglycans.

The use of antioxidants or free-radical scavengers in cosmetic compositions is adequately known per se. Thus, the use of the antioxidative vitamin E in sunscreen formulations is usual. Nevertheless, the effect achieved is even here well short of the hoped-for effect.

Vitamin A and vitamin-A derivatives, such as retinoic acid, retinol and retinol esters, act on the differentiation of epithelial cells and are therefore employed for the prophylaxis and treatment of numerous phenomena which impair the skin state, for example use against acne, psoriasis, senile keratosis, skin discoloration and wrinkles has been described (cf., for example, WO 93/19743 and WO 02/02074).

However, a skin-irritant effect of retinol and derivatives is also described in the literature (for example WO 94/07462). These side effects restrict the use of retinol to narrowly limited areas, it being necessary to avoid overdosing. There is therefore a demand for active ingredients which have a retinol-like spectrum of action, but do not have the side effects described or at least only do so in reduced form.

It has been previously described that compounds acting via "PPAR activation" may be usable as anti-ageing agents.

It has been shown that PPAR-alpha agonists are responsible for epidermal differentiation (increased levels of filaggrin and involucrin). PPAR ligands like oleic acid, linoleic acid and clofibrate accelerated the development of the stratum corneum and epidermal barrier in fetal skin explants derived from rats [G. Weindl et al., Drugs 2005, 65 (14), 1919-1934].

PPAR-alpha agonists causes reduced inflammation via the reduction of interleukin-1alpha (IL-1a), also PPAR-alpha-induced reduction of IL-1alpha could result in less melanin being produced and thus incorporated per cell [J. W. Wiechers et al., International Journal of Cosmetic Science, 2005, 27, 123-132].

PPAR activators like Clofibrate increases the expression of profilaggrin, a major constituent of keratohyalin granules, increases the processing of profilaggrin to filaggrin, and increases the expression of loricrin, a key structural protein of the cornified envelope. Furthermore they induced differentiation in human keratinocyte cultures, as indicated by increased protein and mRNA levels of two differentiation-specific proteins: involucrin and transglutaminase. Similar to other inducers of differentiation, such as vitamin D derivates and retinoic acid, clofibrate inhibits keratinocyte growth and proliferation in vitro and in vivo [Hanley K, et al., J Invest Dermatol 1998 Apr; 110 (4): 368-75 and Komuves LG, J Invest Dermatol 2000 Sep; 115 (3): 353-60] PPAR-alpha might be important for the development of the epidermis during late embryogenesis, but dispensable for renewal of the epidermis in the adult animal [L. Michalik, W. Wahli,, Biochimica et Biophysica Acta 1771 (2007) 991-998].

Interestingly, the PPAR-alpha agonist clofibrate, within a narrow range of concentrations, increases the survival of human hair follicles [L. Michalik, W. Wahli, Biochimica et Biophysica Acta 1771 (2007) 991-998].

More recently, PPAR activators (PPAR-alpha: clofibrate; PPAR-beta/delta; GW501516; PPAR-gamma: ciglitazone, troglitazone or GI262570) were reported to increase the expression of a key enzyme of keratinocyte differentiation and epidermal barrier maturation, namely, the cholesterol sulfotransferase type 2B1b[L. Michalik, W. Wahli,, Biochimica et Biophysica Acta 1771 (2007) 991-998].

PPAR-alpha favours skin healing via modulation of the inflammatory phase, while PPAR-beta is an important player of keratinocyte survival and migration.

Consistently, transgenic mice overexpressing PPAR-alpha in the epidermis show reduced hyperplasia upon topical application of TPA, suggesting that PPAR-alpha prevents hyperproliferation of keratinocytes in adult mouse skin [Q. Yang et al, J. Invest. Dermatol. 126 (2006) 374-385.]

Reduced inflammation via the reduction of interleukin-1alpha (IL-a), therefore is skin tanning as a reaction to UV-induced irradiation. In the short term, upregulating the p38 pathway leads to increased tyrosinase production. In the long-term frame, inflammatory cytokines such as IL-alpha act in a communicative way on melanocytes that also increases tyrosinase levels. PPAR-alpha-induced reduction of IL-1alpha could therefore result in less melanin being produced and thus incorporated per cell[J. W. Wiechers et al., International Journal of Cosmetic Science, 2005, 27, 123-132] PPAR gamma activators inhibit melanoma proliferation in a dose-dependent manner (reduced tyrosinase production and/or a reduced tyrosinase half-life.

PPAR-alpha and -gamma are upregulated in the interfollicular epidermis of mice during the wound healing or proliferation induced by topical application of 12-o-tetradecanoyl-phorbol-13-acetate (TPA) [G. Weindl et al., Drugs 2005,

65 (14), 1919-1934,].

TNF-α released by injured epidermal keratinocytes activates stress-associated protein kinase (SAPK) and induces AP-1 binding to the PPARδ (PPAR-delta or PPAR-beta) promoter and transcription of PPARδ target genes. TNF-α also triggers production of endogenous PPARδ ligands, which activate PPARδ in keratinocytes and macrophages.

PPARδ activation helps maintain a sufficient number of keratinocytes for re-epithelialization by improving apoptosis resistance through expression of integrin-linked kinase (ILK) and 3-phosphoinositide-dependent kinase (PDK), as well as via activation of the PKB/Akt-1 survival pathway. The initial inflammatory signals that stimulate PPARδ are countered by TGF-β1/Smad3-mediated suppression of PPARδ in the late re-epithelialization/remodeling stage. This suppression occurs via Smad3/4 complex-mediated abrogation of AP-1 activity. In addition, TGF-β1 released by dermal wound fibroblasts increases macrophage numbers and stimulates ECM production for wound remodelling [L. Michalik, W. Wahli,, Biochimica et Biophysica Acta 1771 (2007) 991-998]

PPAR activators allow a new mechanism of action for skin lightening and/or whitening. Activators of PPAR receptor are known as a skin whitening agent. Binding of activators to PPAR-gamma may reduced the melanogenesis. Binding to PPAR-gamma in turn results in less tyrosinase being formed. This in turn leads to reduced melanogenesis both in vitro and in vivo because the compound binds not only to PPAR-gamma but also to PPAR-alpha.

Binding of ligand to the nuclear receptor PPAR-gamma may lead to release of BCL-6, a transcriptional repressor of inflammation. This may result in decreased expression of inflammatory cytokine genes, reduced inflammation, and a decrease in atherosclerosis [L. Michalik, W. Wahli,, Biochimica et Biophysica Acta 1771 (2007) 991-998]

Cosmetic peroxisome proliferator-activated receptor (PPAR) lipids have been shown to significantly improve the photoaged appearance of skin, versus a vehicle effect, and, in combination with glycolic acid, versus glycolic acid alone [J. W. Wiechers et al., International Journal of Cosmetic Science, 2005, 27, 123-132.

However, the compounds known in the art, which are acting via PPAR receptors and used in the cosmetic field as skin whitener like Octadecene Dioic Acid are mainly acting via PPAR-gamma activation (O.D.A White™ (SEDERMA Patents: WO94/07837; US 5,753,704; EP 0 662 946). Up to now no cosmetic product is on the market, which contains a PPAR activator having a balanced PPAR-alpha and PPAR-gamma activity.

The Compound for use according to the instant invention is a novel active ingredient for skin lightening and/or skin whitening.

Preferably, the Compound for use according to the invention has advantageous properties, such as improved handling properties, improved stability properties and/or an advantageous profile of activation of PPAR-alpha and -gamma receptors.

Surprisingly it has been found that the Compound provides a well-balanced stimulation of PPAR-alpha and -gamma receptors, which is in contrast to the other PPAR activators known in the art, which are mostly stimulating either the alpha or gamma receptors or have, at least, a pre-dominant activity on one of such receptors (see, for example, PPAR stimulation by fenofibrate (PPAR-alpha; WY14643), troglitazone (PPAR-gamma; Tontonoz P, Hu E, Spiegelman BM, Cell 1994; 79, 1147-1156) and rosiglitazone (PPAR-gamma, Jo A. Van Ginderachter et al, Blood, 2006, Vol. 108, No. 2, pp. 525-535)□ and Octadecene Dioic Acid (PPAR-gamma, WO94/07837). The very similar simultaneous activation of both PPAR-alpha and -gamma receptors activation in the same concentration range results in beneficial effects for its use in the cosmetic field.

Sertzing and colleagues' observation suggests the possibility that PPAR-alpha and PPAR-gamma activators, or compounds that positively regulate PPAR gene expression, may represent novel Non-steroidal anti-inflammatory drugs for the topical or systemic treatment of common inflammatory skin diseases such as atopic dermatitis, psoriasis, and allergic contact dermatitis (Sertzing P. et al., American Journal of Clinical Dermatology, 2008).

PPAR-alpha is involved in the regulation of fatty acid (FA) uptake and oxidation, inflammation and vascular function, while PPAR-gamma participates in FA uptake and storage, glucose homeostasis and inflammation. To date, a medication that may combine the beneficial metabolic effects of PPAR-alpha and PPAR-gamma activation with fewer undesirable side effects has not been successfully developed (Pomegranate flower: a unique traditional antidiabetic medicine with dual PPAR-alpha/-gamma activator properties. Li Y, Qi Y, Huang TH, Yamahara J, Roufogalis BD, Diabetes, obesity & metabolism (2008).

PPARs are expressed in human melanocytes and PPAR-alpha and PPAR-gamma activators inhibit melanocyte growth and stimulate melanogenesis. Activators for PPAR-alpha (WY-14643) and PPAR-gamma (ciglitazone) inhibited proliferation of melanocytes in a dose-dependent manner, whereas bezafibrate, a preferential activator for PPAR-beta/delta, had no effect (Kang HY et al, Br J Dermatol. 2004 Mar;150(3):462-8.)

Octadecenedioic acid (O.D.A White) is known as a skin whitening agent but its activity is not mediated via a direct inhibition of tyrosinase. Binding to PPAR-gamma results in reduced tyrosinase mRNA expression which in turn results in less tyrosinase being formed. This in turn leads to reduced melanogenesis both in vitro and in vivo Because octadecenedioic acid binds not only to PPAR-gamma but also to PPAR-alpha and PPAR-delta, other efficacies mediated via these receptors may also be expected (Wiechers, J. W. , International Journal of Cosmetic Science. 27(2):123-132, April 2005.). Nevertheless the PPAR-alpha activation of O.D.A. White is negligible (personel communication Johann

Wiechers at Merck KGaA). EMD 336340 has unlike O.D.A. White a balanced activity to both alpha and gamma PPAR receptors.

It is known that PPAR-gamma agonists are involved in adipogenesis [Am J Physiol Endocrinol Metab 293:E1159-E1168, 2007]. It is known that adipocytes are located around hair follicles in the fetal pig. The relationship between hair follicle development and adipocyte formation in the pig hypodermis was studied by Hausman GJ et al [J. Animal Sci. 1982, 54:1286-1296]. They show that the development of hair follicle adipose lobules in the pig is associated temporally and spatially with hair follicle and sweat gland growth and development [J. Animal Sci. 1982, 54:1286-1296].

Adipose tissue also serves as an important endocrine organ (Kershaw EE; J. Clin. Endocrinol. Metab. 89-6: 2548-56, 2004) by producing hormones such as leptin, resistin and the cytokine TNFa. The formation of adipose tissue appears to be controlled by the adipose gene.

It has been found for the Compound that FABP4 (fatty acid binding protein 4: Adipocyte) and Leptin, which are involved in adipogenesis and promotion of fat cells, are significantly upregulated in cDNA microarray data with treated EMD336340 [see results in Appendix A]. LEP (leptin) may function as part of a signaling pathway that acts to regulate the size of the body fat depot. An increase in the level of LEP may act directly or indirectly on the CNS to inhibit food intake and/or regulate energy expenditure as part of a homeostatic mechanism to maintain constancy of the adipose mass. Accordingly the Compound is involved in fat cells modulation and so, in hair follicle development.

The Compound is in particular useful as skin-whitening active in dermatological and cosmetic compositions (alone or in combination with other actives). Therefore, the invention relates to the use of the Compound as skin lightening and/or skin whitening agent.

The Compound has been tested in vitro and shown increased expression of epidermal differentiation proteins, which also are known in the literature as indicators of improvement in skin condition and overall skin health [own unpublished results]

Further, it has been found that the Compound is very safe und shows no toxic effect in animal models in comparison to other PPAR activators. The Compound is not genotoxic, no indication of cell proliferation or apoptosis, no carcinogenic risk is expected during clinical development, no skin/eye irritation, no phototoxic, no skin sensitization (in house data available for: acute toxicity studies, repeated dose studies, mutagenicity studies, reproductive toxicology studies, skin sensitization test, phototoxic and photo-allergenic potential, skin/eye irriation test, effects on general behavior and body temperature, ion-channel binding assay).

The Compound can be used as free acid but also as one of its salts with organic or inorganic bases, of the salts formed with metals and in particular alkali, alkaline earth and transition metals (such as sodium, potassium calcium, magnesium or aluminium) or with bases, such as ammonia or secondary or tertiary amines (such as diethylamine, triethylamine, piperidine, piperazine or morpholine), or with basic amino acids or with osamines (such as meglumine) or with aminoalcohols (such as 3-aminobutanol and 2-aminoethanol).

The term "dermatologically acceptable", as used herein, preferably means that the composition or components described suitable for use in contact with human skin without risk of toxicity, incompatibility instability, allergic response, and the like.

All terms such as "skin ageing", "signs of skin ageing", "topical application", and the like are preferably used in the sense in which they are generally and widely used in the art of developing, testing and marketing cosmetic and personal care products.

The term "cosmetic composition" or preferably more briefly just "composition" in accordance with the present invention preferably relates to a formulation that can be used for cosmetic purposes, purposes of hygiene and/or as a basis for delivery of one or more pharmaceutical ingredients. It is also possible that these formulations are used for two or more of these purposes at one time. Thus, the terms "cosmetics," "cosmetic composition" and/or "composition"as used herein, preferably include without limitation, lipstick, mascara, rouge, foundation, blush, eyeliner, lipliner, lip gloss, facial or body powder, sunscreens and blocks, nail polish, mousse, sprays, styling gels, nail conditioner, whether in the form of creams, lotions, gels, ointments, emulsions, colloids, solutions, suspensions, compacts, solids, pencils, spray-on formulations, brush-on formulations and the like. "Personal care products" preferably include, without limitation, bath and shower gels, shampoos, conditioners, cream rinses, hair dyes and coloring products, leave-on conditioners, sunscreens and sunblocks, lip balms, skin conditioners, cold creams, moisturizers, hair sprays, soaps, body scrubs, exfoliants, astringents, depilatories and permanent waving solutions, antidandruff formulations, antisweat and antiperspirant compositions, shaving, preshaving and after shaving products, moisturizers, deodorants, cold creams, cleansers, skin gels, rinses, whether in solid, powder, liquid, cream, gel, ointment, lotion, emulsions, colloids, solutions, suspensions, or other form.

Compositions in accordance with the invention preferably include cosmetics and personal care products.

Some of the compositions may also provide additional benefits, including stability of the formulation, absence of significant (consumer-unacceptable) skin irritation, anti-inflammatory activity and good aesthetics.

The Compound is contained in said composition in an amount of 0,00001 per cent by weight to 10 per cent by weight, preferably in an amount of 0,001 per cent by weight to 10 per cent by weight, more preferably in an amount of

0,1 per cent by weight to 10 per cent by weight, even more preferably 0,001 per cent by weight to 5 per cent per weight and especially 0,1 per cent by weight to 5 per cent by weight.

A composition may comprise the Compound and at least one further skin-care ingredient and at least one carrier which is suitable for topical applications. Preferably, the Compound and the compositions containing it preferably appear to have a benefit in tissue regeneration. This is believed to be due to their ability to modulate and preferably stimulate the production of certain advantageous biomolecules, including, but not limited to, collagen I, fibronectin, collagen IV and/or hyaluronic acid, in skin cells.

The compositions generally comprise the Compound and one or more vehicle or carrier, preferably one or more cosmetically acceptable vehicle or carrier. The one or more vehicles or carriers may be independently selected from the group of hydrophobic and hydrophilic vehicles or carriers. Suitable, hydrophobic vehicles or carriers include, for example, waxy non-ionic substances, preferably waxy non-ionic substances commonly used in cosmetics, including, but not limited to esters and ethers of fatty alcohols and of fatty acids, with carbon chain length from C4 to C22, preferably from C8 to C18, and most preferably from C-12 to C18- Examples of a fatty hydrophobic carriers or vehicles are preferably selected from the group consisting of isopropyl myristate, isopropyl palmitate, octyl palmitate, isopropyl lanolate, acetylated lanolin alcohol, the benzoate of C12-C15 alcohols, cetearyl octanoate, cetyl palmitate, myristyl myristate, myristyl lactate, cetyl acetate, propylene glycol dicaprylate/caprate, decyl oleate, acetylated lanolin, stearyl heptanoate, diisostearyl malate, octyl hydroxystearate, octyl hydroxystearate, and isopropyl isostearate, and the like. Examples of hydrophilic carrier or vehicles, especially for solutions, are preferably selected from the group consisting of glycols and alkoxylated glycols, preferably glycols and alkoxylated glycols commonly used in cosmetics, including, but not limited to, ethylene glycol, diethylene glycol, triethylene glycol, propylene glycol, dipropylene glycol, and the like.

The compositions, especially the cosmetic compositions may be formulated as creams, lotions, serums, sprays, sticks and other forms known to those skilled in the art. Creams and lotions are the currently preferred product forms. Preferably, the concentration of the Compound in the cosmetically acceptable vehicle may range from 1 ppb to 10,000 ppm, preferably from 10 ppb to 1,000 ppm, more preferably from 100 ppb to 100 ppm, and most preferably from 1 ppm to 100 ppm.

Preferably, cosmetic compositions can typically comprise the carrier solution described above at levels between about 0.01% and about 90% by weight, preferably between about 0.1 % and about 50%, more preferably between about 0.1 % and about 20%, and more preferred still between about 1% and about 10% by weight.

Preferably, the concentration of the Compound in the composition for application to the skin may range from 1 ppb to 10,000 ppm, preferably from 10 ppb to 1,000 ppm, more preferably from 100 ppb to 100 ppm, even more preferably from 0.5 ppm to 150 ppm and most preferably 1 ppm to 100 ppm, for example about 0.5 ppm, about 1 ppm, about 1.5 ppm, about 5 ppm, about 10 ppm, about 25 ppm, about 50 ppm, about 75 ppm, about 100 ppm or about 125 ppm.

If applicable, ppb and ppm preferably are to be regarded to be based on the respective weights, such as of the weight of the respective components (e.g. Compound, vehicle) and/or the weight of the respective component and the total weight of the composition. Accordingly, 1 ppm is preferably regarded as 1 mg/kg or $10^{-4}$ % by weight.

Optionally, the compositions may optionally comprise additional active and inactive ingredients other than the Compound, including, but not limited to, excipients, fillers, emulsifying agents, antioxidants, surfactants, film formers, chelating agents, gelling agents, thickeners, emollients, humectants, moisturizers, vitamins, minerals, viscosity and/or rheology modifiers, sunscreens, keratolytics, depigmenting agents, retinoids, hormonal compounds, alpha-hydroxy acids, alpha-keto acids, anti-mycobacterial agents, antifungal agents, antimicrobials, antivirals, analgesics, lipidic compounds, anti- allergenic agents, H1 and/or or H2 antihistamines, anti-inflammatory agents, anti-irritants, antineoplastics, immune system boosting agents, immune system suppressing agents, anti-acne agents, anesthetics, antiseptics, insect repellents, skin cooling compounds, skin protectants, skin penetration enhancers, exfollients, lubricants, fragrances, colorants, staining agents, depigmenting agents, hypopigmenting agents, preservatives, stabilizers, pharmaceutical agents, photostabilizing agents, and mixtures thereof. In addition to the foregoing, the personal care products may contain any other compound for the treatment of skin disorders.

The compositions here are non-therapeutical, more preferably either compositions which can be used topically, for example cosmetic or dermatological formulations, or foods or food supplements. In this case, the compositions comprise a cosmetically or dermatologically or food-suitable carrier and, depending on the desired property profile, optionally further suitable ingredients.

The Compound is typically employed in amounts of from 0.01 to 20% by weight, preferably in amounts of from 0.1 % by weight to 10% by weight and particularly preferably in amounts of from 1 to 8% by weight. The person skilled in the art has absolutely no difficulties in selecting the amount correspondingly depending on the intended action of the composition.

The composition may further comprise antioxidants having protective action against oxidative stress or against the effect of free radicals, where the person skilled in the art has absolutely no difficulties in selecting antioxidants having a suitably fast or time-delayed action. There are many proven substances known from the specialist literature which can be used as antioxidants, for example amino acids (for example glycine, histidine, tyrosine, tryptophan) and derivatives

thereof, imidazoles (for example urocanic acid) and derivatives thereof, peptides, such as D,L-carnosine, D-carnosine, L-carnosine and derivatives thereof (for example anserine), carotinoids, carotenes (for example α-carotene, β-carotene, lycopene) and derivatives thereof, chlorogenic acid and derivatives thereof, lipoic acid and derivatives thereof (for example dihydrolipoic acid), aurothioglucose, propylthiouracil and other thiols (for example thioredoxin, glutathione, cysteine, cystine, cystamine and the glycosyl, N-acetyl, methyl, ethyl, propyl, amyl, butyl and lauryl, palmitoyl, oleyl, γ-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts), and sulfoximine compounds (for example buthionine sulfoximines, homocysteine sulfoximine, buthionine sulfones, penta-, hexa- and heptathionine sulfoximine) in very low tolerated doses (for example pmol to μmol/kg), furthermore (metal) chelating agents (for example α-hydroxy fatty acids, palmitic acid, phytic acid, lactoferrin), α-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof, vitamin C and derivatives (for example ascorbyl palmitate, magnesium ascorbyl phosphate, ascorbyl acetate), tocopherols and derivatives (for example vitamin E acetate), and coniferyl benzoate of benzoin resin, rutinic acid and derivatives thereof, α-glycosylrutin, ferulic acid, furfurylideneglucitol, carnosine, butylhydroxytoluene, butylhydroxyanisole, nordihydroguaiaretic acid, trihydroxybutyrophenone, quercetin, uric acid and derivatives thereof, mannose and derivatives thereof, zinc and derivatives thereof (for example ZnO, $ZnSO_4$), selenium and derivatives thereof (for example selenomethionine), stilbenes and derivatives thereof (for example stilbene oxide, trans-stilbene oxide).

Suitable antioxidants are furthermore described in WO 2006/111233 and WO 2006/111234.

Suitable antioxidants are also compounds of the general formula A and/or B

A 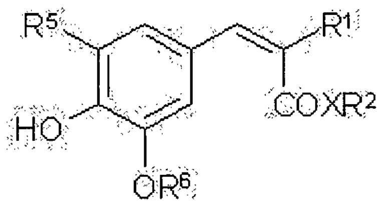 oder 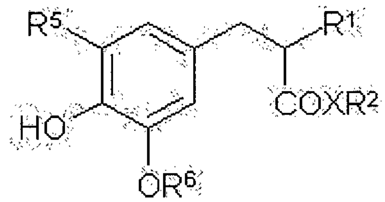 B

,

wherein

$R^1$ is selected from the group consisting of $-C(O)CH_3$, $-CO_2R^3$, $-C(O)NH_2$ and $-C(O)N(R^4)_2$,

X is O or NH,

$R^2$ is linear or branched Alkyl having 1 to 30 C-atoms,

$R^3$ is linear or branched Alkyl having 1 to 20 C-atoms,

$R^4$ is in each case independently selected from the group consisting of H and linear or branched Alkyl having 1 to 8 C-atoms,

$R^5$ is selected from the group consisting of linear or branched Alkyl having 1 to 8 C-atoms and linear or branched Alkoxy having 1 to 8 C-atoms and

$R^6$ is selected from the group consisting of linear or branched Alkyl mit 1 to 8 C-atoms bedeutet, preferably selected from derivatives of the 2-(4-Hydroxy-3,5-dimethoxybenzyliden)-malonic acid and/or 2-(4-Hydroxy-3,5-dimethoxybenzyl)-malonic acid , and especially preferably selected from 2-(4-Hydroxy-3,5-dimethoxybenzyliden)-malonic acid-bis-(2-ethylhexyl)ester (z.B. Oxynex® ST Liquid) and/or 2-(4-Hydroxy-3,5-dimethoxybenzyl)-malonic acid-bis-(2-ethylhexyl)ester (z.B. RonaCare® AP).

Mixtures of antioxidants are likewise suitable for use in the cosmetic compositions. Known and commercial mixtures are, for example, mixtures comprising, as active ingredients, lecithin, L-(+)-ascorbyl palmitate and citric acid (for example Oxynex® AP), natural tocopherols, L-(+)-ascorbyl palmitate, L-(+)-ascorbic acid and citric acid (for example Oxynex® K LIQUID), tocopherol extracts from natural sources, L-(+)-ascorbyl palmitate, L-(+)-ascorbic acid and citric acid (for example Oxynex® L LIQUID), DL-α-tocopherol, L-(+)-ascorbyl palmitate, citric acid and lecithin (for example Oxynex® LM) or butylhydroxytoluene (BHT), L-(+)-ascorbyl palmitate and citric acid (for example Oxynex® 2004). Antioxidants of this type are usually employed with the Compound in compositions of this type in ratios in the range from 1000:1 to 1:1000, preferably in amounts of from 100:1 to 1:100.

The compositions may comprise vitamins as further ingredients. The cosmetic compositions preferably comprise vitamins and vitamin derivatives selected from vitamin B, thiamine chloride hydrochloride (vitamin $B_1$), riboflavin (vitamin $B_2$), nicotinamide, vitamin C (ascorbic acid), vitamin D, ergocalciferol (vitamin $D_2$), vitamin E, DL-$\alpha$-tocopherol, tocopherol E acetate, tocopherol hydrogensuccinate, vitamin $K_1$, esculin (vitamin P active ingredient), thiamine (vitamin $B_1$), nicotinic acid (niacin), pyridoxine, pyridoxal, pyridoxamine (vitamin $B_6$), pantothenic acid, biotin, folic acid and cobalamine (vitamin $B_{12}$), particularly preferably vitamin C and derivatives thereof, DL-$\alpha$-tocopherol, tocopherol E acetate, nicotinic acid, pantothenic acid and biotin. Vitamins are usually employed here with the Compound, in ratios in the range from 1000:1 to 1:1000, preferably in amounts of from 100:1 to 1:100.

Of the phenols having an antioxidative action, the polyphenols, some of which are naturally occurring, are of particular interest for applications in the pharmaceutical, cosmetic or nutrition sector. For example, the flavonoids or bioflavonoids, which are principally known as plant dyes, frequently have an antioxidant potential. K. Lemanska, H. Szymusiak, B. Tyrakowska, R. Zielinski, I.M.C.M. Rietjens; Current Topics in Biophysics 2000, 24(2), 101-108, are concerned with effects of the substitution pattern of mono- and dihydroxyflavones. It is observed therein that dihydroxyflavones containing an OH group adjacent to the keto function or OH groups in the 3',4'- or 6,7- or 7,8-position have antioxidative properties, while other mono- and dihydroxyflavones in some cases do not have antioxidative properties.

Quercetin (cyanidanol, cyanidenolon 1522, meletin, sophoretin, ericin, 3,3',4',5,7-pentahydroxyflavone) is frequently mentioned as a particularly effective antioxidant (for example C.A. Rice-Evans, N.J. Miller, G. Paganga, Trends in Plant Science 1997, 2(4), 152-159). K. Lemanska, H. Szymusiak, B. Tyrakowska, R. Zielinski, A.E.M.F. Soffers, I.M.C.M. Rietjens; Free Radical Biology&Medicine 2001, 31(7), 869-881, have investigated the pH dependence of the antioxidant action of hydroxyflavones. Quercetin exhibits the greatest activity amongst the structures investigated over the entire pH range.

Suitable antioxidants are furthermore compounds of the formula II

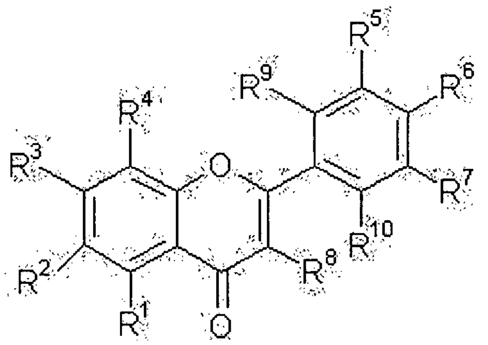

II

where $R^1$ to $R^{10}$ may be identical or different and are selected from

- H
- $OR^{11}$
- straight-chain or branched $C_1$- to $C_{20}$-alkyl groups,
- straight-chain or branched $C_3$- to $C_{20}$-alkenyl groups,
- straight-chain or branched $C_1$- to $C_{20}$-hydroxyalkyl groups, where the hydroxyl group may be bonded to a primary or secondary carbon atom of the chain and furthermore the alkyl chain may also be interrupted by oxygen, and/or
- $C_3$- to $C_{10}$-cycloalkyl groups and/or $C_3$- to $C_{12}$-cycloalkenyl groups, where the rings may each also be bridged by $-(CH_2)_n-$ groups, where n = 1 to 3,
- where all $OR^{11}$ are, independently of one another,
  - OH
  - straight-chain or branched $C_1$- to $C_{20}$-alkoxy groups,

- straight-chain or branched $C_3$- to $C_{20}$-alkenyloxy groups,

- straight-chain or branched $C_1$- to $C_{20}$-hydroxyalkoxy groups, where the hydroxyl group(s) may be bonded to a primary or secondary carbon atom of the chain and furthermore the alkyl chain may also be interrupted by oxygen, and/or

- $C_3$- to $C_{10}$-cycloalkoxy groups and/or $C_3$- to $C_{12}$-cycloalkenyloxy groups, where the rings may each also be bridged by $-(CH_2)_n-$ groups, where n = 1 to 3, and/or

- mono- and/or oligoglycosyl radicals,

with the proviso that at least 4 radicals from $R^1$ to $R^7$ are OH and that the molecule contains at least two pairs of adjacent -OH groups,

- or $R^2$, $R^5$ and $R^6$ are OH and the radicals $R^1$, $R^3$, $R^4$ and $R^{7-10}$ are H,

as described in the earlier German patent application DE 10244282.7.

Besides the advantages mentioned above, the advantages of the compositions comprising at least one antioxidant here are, in particular, the antioxidant action and the good skin tolerability. In addition, the compounds described here are preferably colourless or have only a weak colour and thus only result in slight discoloration of the compositions, or none at all.

Of particular advantage are non-therapeutic compositions, comprising at least one compound of the formula II which is characterised in that at least two adjacent radicals of the radicals $R^1$ to $R^4$ are OH and at least two adjacent radicals of the radicals $R^5$ to $R^7$ are OH. Particularly preferred compositions comprise at least one compound of the formula II which is characterised in that at least three adjacent radicals of the radicals $R^1$ to $R^4$ are OH, preferably with the radicals $R^1$ to $R^3$ being OH.

It is also advantageous to administer the compounds of the formula II in encapsulated form, for example as cellulose or chitin capsules, in gelatine or wax matrices or encapsulated with cyclodextrins.

Compositions which are particularly preferred also comprise UV filters besides the Compound.

In principle, all UV filters are suitable for combination with the Compound. Particular preference is given to UV filters whose physiological acceptability has already been demonstrated. Both for UVA and UVB filters, there are many proven substances which are known from the specialist literature, for example:

benzylidenecamphor derivatives, such as 3-(4'-methylbenzylidene)-dl-camphor (for example Eusolex® 6300), 3-benzylidenecamphor (for example Mexoryl® SD), polymers of N-{(2 and 4)-[(2-oxoborn-3-ylidene)methyl]benzyl}acrylamide (for example Mexoryl® SW), N,N,N-trimethyl-4-(2-oxoborn-3-ylidene-methyl)anilinium methylsulfate (for example Mexoryl® SK) or (2-oxoborn-3-ylidene)toluene-4-sulfonic acid (for example Mexoryl® SL),

benzoyl- or dibenzoylmethanes, such as 1-(4-tert-butylphenyl)-3-(4-methoxy-phenyl)propane-1,3-dione (for example Eusolex® 9020) or 4-isopropyldibenzoyl-methane (for example Eusolex® 8020),

benzophenones, such as 2-hydroxy-4-methoxybenzophenone (for example Eusolex® 4360) or 2-hydroxy-4-methoxybenzophenone-5-sulfonic acid and its sodium salt (for example Uvinul® MS-40),

methoxycinnamic acid esters, such as octyl methoxycinnamate (for example Eusolex® 2292) or isopentyl 4-methoxycinnamate, for example as a mixture of the isomers (for example Neo Heliopan® E 1000),

salicylate derivatives, such as 2-ethylhexyl salicylate (for example Eusolex® OS), 4-isopropylbenzyl salicylate (for example Megasol®) or 3,3,5-trimethylcyclohexyl salicylate (for example Eusolex® HMS),

4-aminobenzoic acid and derivatives, such as 4-aminobenzoic acid, 2-ethylhexyl 4-(dimethylamino)benzoate (for example Eusolex® 6007) or ethoxylated ethyl 4-aminobenzoate (for example Uvinul® P25),

phenylbenzimidazolesulfonic acids, such as 2-phenylbenzimidazole-5-sulfonic acid and potassium, sodium and triethanolamine salts thereof (for example Eusolex® 232), 2,2-(1,4-phenylene)bisbenzimidazole-4,6-disulfonic acid and salts thereof (for example Neoheliopan® AP) or 2,2-(1,4-phenylene)bisbenz-imidazole-6-sulfonic acid;

and further substances, such as

- 2-ethylhexyl 2-cyano-3,3-diphenylacrylate (for example Eusolex® OCR),
- 3,3'-(1,4-phenylenedimethylene)bis(7,7-dimethyl-2-oxobicyclo[2.2.1]hept-1-ylmethanesulfonic acid and salts thereof (for example Mexoryl® SX),
- 2,4,6-trianilino-(p-carbo-2'-ethylhexyl-1'-oxy)-1,3,5-triazine (for example Uvinul® T 150) and
- hexyl 2-(4-diethylamino-2-hydroxybenzoyl)benzoate (for example Uvinul® UVA Plus, BASF).

The compounds mentioned in the list should only be regarded as examples. It is of course also possible to use other UV filters.

These organic UV filters are generally incorporated into cosmetic formulations in an amount of from 0.5 to 10 per cent by weight, preferably 1 - 8%.

Further suitable organic UV filters are, for example,

- 2-(2H-benzotriazol-2-yl)-4-methyl-6-(2-methyl-3-(1,3,3,3-tetramethyl-1-(trimethylsilyloxy)disiloxanyl)propyl)phenol (for example Silatrizole®),
- 2-ethylhexyl 4,4'-[(6-[4-((1,1-dimethylethyl)aminocarbonyl)phenylamino]-1,3,5-triazine-2,4-diyl)diimino]bis(benzoate) (for example Uvasorb® HEB),
- $\alpha$-(trimethylsilyl)-$\omega$-[trimethylsilyl)oxy]poly[oxy(dimethyl [and about 6% of methyl[2-[p-[2,2-bis(ethoxycarbonyl]vinyl]phenoxy]-1-methyleneethyl] and approximately 1.5% of methyl[3-[p-[2,2-bis(ethoxycarbonyl)vinyl]phenoxy]-propenyl] and from 0.1 to 0.4% of (methylhydrogen]silylene]] ($n \approx 60$) (CAS No. 207 574-74-1)
- 2,2'-methylenebis(6-(2H-benzotriazol-2-yl)-4-(1,1,3,3-tetramethylbutyl)-phenol) (CAS No. 103 597-45-1)
- 2,2'-(1,4-phenylene)bis(1H-benzimidazole-4,6-disulfonic acid, monosodium salt) (CAS No. 180 898-37-7),
- 2,4-bis{[4-(2-ethylhexyloxy)-2-hydroxy]phenyl}-6-(4-methoxyphenyl)-1,3,5-triazine (CAS No. 103 597-45-, 187 393-00-6) and
- 2-ethylhexyl 4,4'-[(6-[4-((1,1-dimethylethyl)aminocarbonyl)phenylamino]-1,3,5-triazine-2,4-diyl)diimino]bis(benzoate) (for example Uvasorb® HEB).

Further suitable UV filters are also methoxyflavones corresponding to the earlier German patent application DE 10232595.2.

Organic UV filters are generally incorporated into cosmetic formulations in an amount of from 0.5 to 20 per cent by weight, preferably 1 - 15%.

Conceivable inorganic UV filters are those from the group consisting of titanium dioxides, such as, for example, coated titanium dioxide (for example Eusolex® T-2000, Eusolex® T-AQUA), zinc oxides (for example Sachtotec®), iron oxides and also cerium oxides. These inorganic UV filters are generally incorporated into cosmetic compositions in an amount of from 0.5 to 20 per cent by weight, preferably 2-10%.

Preferred compounds having UV-filtering properties are 3-(4'-methylbenzyl-idene)-dl-camphor, 1-(4-tert-butylphenyl)-3-(4-methoxyphenyl)propane-1,3-dione, 4-isopropyldibenzoylmethane, 2-hydroxy-4-methoxybenzophenone, octyl methoxycinnamate, 3,3,5-trimethylcyclohexyl salicylate, 2-ethylhexyl 4-(dimethylamino)benzoate, 2-ethylhexyl 2-cyano-3,3-diphenylacrylate, 2-phenylbenzimidazole-5-sulfonic acid and its potassium, sodium and triethanolamine salts.

Through combination of the Compound with further UV filters, the protective action against harmful influences of UV radiation can be optimised.

Optimised compositions may comprise, for example, the combination of the organic UV filters 4'-methoxy-6-hydroxyflavone with 1-(4-tert-butylphenyl)-3-(4-methoxyphenyl)propane-1,3-dione and 3-(4'-methylbenzylidene)-dl-camphor. This combination gives rise to broad-band protection, which can be supplemented by the addition of inorganic UV filters, such as titanium dioxide micro-particles.

All the said UV filters can also be employed in encapsulated form. In particular, it is advantageous to employ organic UV filters in encapsulated form. In detail, the following advantages arise:

- The hydrophilicity of the capsule wall can be set independently of the solubility of the UV filter. Thus, for example, it is also possible to incorporate hydrophobic UV filters into purely aqueous compositions. In addition, the oily

impression on application of the composition comprising hydrophobic UV filters, which is frequently regarded as unpleasant, is suppressed.

- Certain UV filters, in particular dibenzoylmethane derivatives, exhibit only reduced photostability in cosmetic compositions. Encapsulation of these filters or compounds which impair the photostability of these filters, such as, for example, cinnamic acid derivatives, enables the photostability of the entire composition to be increased.

- Skin penetration by organic UV filters and the associated potential for irritation on direct application to the human skin is repeatedly being discussed in the literature. The encapsulation of the corresponding substances which is proposed here suppresses this effect.

- In general, encapsulation of individual UV filters or other ingredients enables composition problems caused by the interaction of individual composition constituents with one another, such as crystallisation processes, precipitation and agglomerate formation, to be avoided since the interaction is suppressed.

It is therefore preferred for one or more of the above-mentioned UV filters to be in encapsulated form. It is advantageous here for the capsules to be so small that they cannot be viewed with the naked eye. In order to achieve the above-mentioned effects, it is furthermore necessary for the capsules to be sufficiently stable and the encapsulated active ingredient (UV filter) only to be released to the environment to a small extent, or not at all.

Suitable capsules can have walls of inorganic or organic polymers. For example, US 6,242,099 B1 describes the production of suitable capsules with walls of chitin, chitin derivatives or polyhydroxylated polyamines. Capsules which can particularly preferably be employed have walls which can be obtained by a sol-gel process, as described in the applications WO 00/09652, WO 00/72806 and WO 00/71084. Preference is again given here to capsules whose walls are built up from silica gel (silica; undefined silicon oxide hydroxide). The production of corresponding capsules is known to the person skilled in the art, for example from the cited patent applications, whose contents expressly also belong to the subject-matter of the present application.

The capsules are preferably present in compositions in amounts which ensure that the encapsulated UV filters are present in the composition in the above-indicated amounts.

The skin-protecting or skin-care active ingredients can in principle be any active ingredients known to the person skilled in the art.

Particularly preferred active ingredients are pyrimidinecarboxylic acids and/or aryl oximes.

Pyrimidinecarboxylic acids occur in halophilic microorganisms and play a role in osmoregulation of these organisms (E.A. Galinski et al., Eur. J. Biochem., 149 (1985) pages 135-139). Of the pyrimidinecarboxylic acids, particular mention should be made here of ectoine ((S)-1,4,5,6-tetrahydro-2-methyl-4-pyrimidinecarboxylic acid) and hydroxyectoine ((S,S)-1,4,5,6-tetrahydro-5-hydroxy-2-methyl-4-pyrimidinecarboxylic acid) and derivatives thereof. These compounds stabilise enzymes and other biomolecules in aqueous solutions and organic solvents. Furthermore, they stabilise, in particular, enzymes against denaturing conditions, such as salts, extreme pH values, surfactants, urea, guanidinium chloride and other compounds.

Ectoine and ectoine derivatives, such as hydroxyectoine, can advantageously be used in medicaments. In particular, hydroxyectoine can be employed for the preparation of a medicament for the treatment of skin diseases. Other areas of application of hydroxyectoine and other ectoine derivatives are typically in areas in which, for example, trehalose is used as additive. Thus, ectoine derivatives, such as hydroxyectoine, can be used as protectant in dried yeast and bacteria cells. Pharmaceutical products, such as non-glycosylated, pharmaceutically active peptides and proteins, for example t-PA, can also be protected with ectoine or its derivatives.

Of the cosmetic applications, particular mention should be made of the use of ectoine and ectoine derivatives for the care of aged, dry or irritated skin. Thus, European patent application EP-A-0 671 161 describes, in particular, that ectoine and hydroxyectoine are employed in cosmetic compositions, such as powders, soaps, surfactant-containing cleansing products, lipsticks, rouge, make-ups, care creams and sunscreen compositions.

Preference is given here to the use of a pyrimidinecarboxylic acid of the following formula III

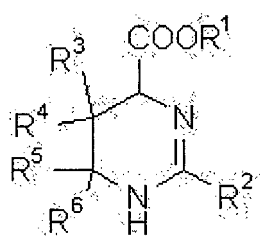

III

in which

| | |
|---|---|
| $R^1$ | is a radical H or C1-8-alkyl, |
| $R^2$ | is a radical H or C1-4-alkyl, and |
| $R^3$, $R^4$, $R^5$ and $R^6$ | are each, independently of one another, a radical from the group consisting of H, OH, $NH_2$ and C1-4-alkyl. Preference is given to the use of pyrimidinecarboxylic acids in which $R^2$ is a methyl or ethyl group, and $R^1$ or $R^5$ and $R^6$ are H. |

Particular preference is given to the use of the pyrimidinecarboxylic acids ectoine ((S)-1,4,5,6-tetrahydro-2-methyl-4-pyrimidinecarboxylic acid) and hydroxyectoine ((S,S)-1,4,5,6-tetrahydro-5-hydroxy-2-methyl-4-pyrimidine-carboxylic acid). The compositions preferably comprise pyrimidinecarboxylic acids of this type in amounts of up to 15% by weight. The pyrimidinecarboxylic acids are preferably employed here in ratios of from 100:1 to 1:100 with respect to the Compound with ratios in the range from 1:10 to 10:1 being particularly preferred.

Of the aryl oximes, preference is given to the use of 2-hydroxy-5-methyllauro-phenone oxime, which is also known as HMLO, LPO or F5. Its suitability for use in cosmetic compositions is disclosed, for example, in DE-A-41 16 123. Compositions which comprise 2-hydroxy-5-methyllaurophenone oxime are accordingly suitable for the treatment of skin diseases which are accompanied by inflammation. It is known that compositions of this type can be used, for example, for the therapy of psoriasis, various forms of eczema, irritative and toxic dermatitis, UV dermatitis and further allergic and/or inflammatory diseases of the skin and integumentary appendages. Compositions which, in addition to the Compound additionally comprise an aryl oxime, preferably 2-hydroxy-5-methyllaurophenone oxime, exhibit surprising antiinflammatory suitability. The compositions here preferably comprise from 0.01 to 10% by weight of the aryl oxime, it being particularly preferred for the composition to comprise from 0.05 to 5% by weight of aryl oxime.

All compounds or components which can be used in the compositions are either known or commercially available or can be synthesised by known processes. The preparation of the Compound is described in US 6,596,758 B1 as Example 16.

The Compound can be incorporated into cosmetic or dermatological compositions in the customary manner. Suitable compositions are those for external use, for example in the form of a cream, lotion or gel or as a solution which can be sprayed onto the skin. Suitable for internal use are administration forms such as capsules, coated tablets, powders, tablet solutions or solutions.

Use forms of the compositions that may be mentioned are, for example, solutions, suspensions, emulsions, PIT emulsions, pastes, ointments, gels, creams, lotions, powders, soaps, surfactant-containing cleansing preparations, oils, aerosols and sprays. Examples of other use forms are sticks, shampoos and shower compositions. Any desired customary carriers, assistants and, if desired, further active ingredients may be added to the composition.

Preferred assistants originate from the group consisting of preservatives, antioxidants, stabilisers, solubilisers, vitamins, colorants and odour improvers.

Ointments, pastes, creams and gels may comprise the customary carriers, for example animal and vegetable fats, waxes, paraffins, starch, tragacanth, cellulose derivatives, polyethylene glycols, silicones, bentonites, silica, talc and zinc oxide, or mixtures of these substances.

Powders and sprays may comprise the customary carriers, for example lactose, talc, silica, aluminium hydroxide, calcium silicate and polyamide powder, or mixtures of these substances. Sprays may additionally comprise the customary propellants, for example chlorofluorocarbons, propane/butane or dimethyl ether.

Solutions and emulsions may comprise the customary carriers, such as solvents, solubilisers and emulsifiers, for example water, ethanol, isopropanol, ethyl carbonate, ethyl acetate, benzyl alcohol, benzyl benzoate, propylene glycol, 1,3-butyl glycol, oils, in particular cottonseed oil, peanut oil, wheatgerm oil, olive oil, castor oil and sesame oil, glycerol fatty acid esters, polyethylene glycols and fatty acid esters of sorbitan, or mixtures of these substances.

Suspensions may comprise the customary carriers, such as liquid diluents, for example water, ethanol or propylene glycol, suspending agents, for example ethoxylated isostearyl alcohols, polyoxyethylene sorbitol esters and polyoxyethylene sorbitan esters, microcrystalline cellulose, aluminium metahydroxide, bentonite, agar-agar and tragacanth, or mixtures of these substances.

Soaps may comprise the customary carriers, such as alkali metal salts of fatty acids, salts of fatty acid monoesters, fatty acid protein hydrolysates, isethionates, lanolin, fatty alcohol, vegetable oils, plant extracts, glycerol, sugars, or mixtures of these substances.

Surfactant-containing cleansing products may comprise the customary carriers, such as salts of fatty alcohol sulfates, fatty alcohol ether sulfates, sulfosuccinic acid monoesters, fatty acid protein hydrolysates, isothionates, imidazolinium derivatives, methyl taurates, sarcosinates, fatty acid amide ether sulfates, alkyl-amidobetaines, fatty alcohols, fatty acid glycerides, fatty acid diethanolamides, vegetable and synthetic oils, lanolin derivatives, ethoxylated glycerol fatty acid esters, or mixtures of these substances.

Face and body oils may comprise the customary carriers, such as synthetic oils, such as fatty acid esters, fatty alcohols, silicone oils, natural oils, such as vegetable oils and oily plant extracts, paraffin oils or lanolin oils, or mixtures of these substances.

Further typical cosmetic use forms are also lipsticks, lip-care sticks, mascara, eyeliner, eye-shadow, rouge, powder make-up, emulsion make-up and wax make-up, and sunscreen, pre-sun and after-sun preparations.

The preferred composition forms include, in particular, emulsions.

Emulsions are advantageous and comprise, for example, the said fats, oils, waxes and other fatty substances, as well as water and an emulsifier, as usually used for a composition of this type.

The lipid phase may advantageously be selected from the following group of substances:

- mineral oils, mineral waxes;
- oils, such as triglycerides of capric or caprylic acid, furthermore natural oils, such as, for example, castor oil;
- fats, waxes and other natural and synthetic fatty substances, preferably esters of fatty acids with alcohols having a low carbon number, for example with isopropanol, propylene glycol or glycerol, or esters of fatty alcohols with alkanoic acids having a low carbon number or with fatty acids;
- silicone oils, such as dimethylpolysiloxanes, diethylpolysiloxanes, diphenylpolysiloxanes and mixed forms thereof.

The oil phase of the emulsions, oleogels or hydrodispersions or lipodispersions is advantageously selected from the group consisting of esters of saturated and/or unsaturated, branched and/or unbranched alkanecarboxylic acids having a chain length of from 3 to 30 carbon atoms and saturated and/or unsaturated, branched and/or unbranched alcohols having a chain length of from 3 to 30 carbon atoms, or from the group consisting of esters of aromatic carboxylic acids and saturated and/or unsaturated, branched and/or unbranched alcohols having a chain length of from 3 to 30 carbon atoms. Ester oils of this type can then advantageously be selected from the group consisting of isopropyl myristate, isopropyl palmitate, isopropyl stearate, isopropyl oleate, n-butyl stearate, n-hexyl laurate, n-decyl oleate, isooctyl stearate, isononyl stearate, isononyl isononanoate, 2-ethylhexyl palmitate, 2-ethylhexyl laurate, 2-hexyldecyl stearate, 2-octyldodecyl palmitate, oleyl oleate, oleyl erucate, erucyl oleate, erucyl erucate and synthetic, semi-synthetic and natural mixtures of esters of this type, for example jojoba oil.

The oil phase may furthermore advantageously be selected from the group consisting of branched and unbranched hydrocarbons and waxes, silicone oils, dialkyl ethers, or the group consisting of saturated and unsaturated, branched and unbranched alcohols, and fatty acid triglycerides, specifically the triglycerol esters of saturated and/or unsaturated, branched and/or unbranched alkanecarboxylic acids having a chain length of from 8 to 24, in particular 12-18, carbon atoms. The fatty acid triglycerides may advantageously be selected, for example, from the group consisting of synthetic, semi-synthetic and natural oils, for example olive oil, sunflower oil, soya oil, peanut oil, rapeseed oil, almond oil, palm oil, coconut oil, palm kernel oil and the like.

Any desired mixtures of oil and wax components of this type may also advantageously be employed. It may also be advantageous to employ waxes, for example cetyl palmitate, as the only lipid component of the oil phase.

The oil phase is advantageously selected from the group consisting of 2-ethylhexyl isostearate, octyldodecanol, isotridecyl isononanoate, isoeicosane, 2-ethylhexyl cocoate, $C_{12-15}$-alkyl benzoate, caprylic/capric acid triglyceride and dicapryl ether.

Particularly advantageous are mixtures of $C_{12-15}$-alkyl benzoate and 2-ethylhexyl isostearate, mixtures of $C_{12-15}$-alkyl benzoate and isotridecyl isononanoate, as well as mixtures of $C_{12-15}$-alkyl benzoate, 2-ethylhexyl isostearate and isotridecyl isononanoate.

Of the hydrocarbons, paraffin oil, squalane and squalene may advantageously be used.

Furthermore, the oil phase may also advantageously have a content of cyclic or linear silicone oils or consist entirely of oils of this type, although it is preferred to use an additional content of other oil-phase components in addition to the silicone oil or the silicone oils.

The silicone oil to be used is advantageously cyclomethicone (octamethylcyclotetrasiloxane). However, it is also advantageous to use other silicone oils, for example hexamethylcyclotrisiloxane, polydimethylsiloxane or poly(methylphenylsiloxane).

Also particularly advantageous are mixtures of cyclomethicone and isotridecyl isononanoate and of cyclomethicone and 2-ethylhexyl isostearate.

The aqueous phase of the compositions optionally advantageously comprises alcohols, diols or polyols having a low carbon number, and ethers thereof, preferably ethanol, isopropanol, propylene glycol, glycerol, ethylene glycol, ethylene glycol monoethyl or monobutyl ether, propylene glycol monomethyl, monoethyl or monobutyl ether, diethylene glycol monomethyl or monoethyl ether and analogous products, furthermore alcohols having a low carbon number, for

example ethanol, isopropanol, 1,2-propanediol or glycerol, and, in particular, one or more thickeners, which may advantageously be selected from the group consisting of silicon dioxide, aluminium silicates, polysaccharides and derivatives thereof, for example hyaluronic acid, xanthan gum, hydroxypropylmethylcellulose, particularly advantageously from the group consisting of the polyacrylates, preferably a polyacrylate from the group consisting of the so-called Carbopols, for example Carbopol grades 980, 981, 1382, 2984 or 5984, in each case individually or in combination.

In particular, mixtures of the above-mentioned solvents are used. In the case of alcoholic solvents, water may be a further constituent.

Emulsions are advantageous and comprise, for example, the said fats, oils, waxes and other fatty substances, as well as water and an emulsifier, as usually used for a formulation of this type.

In a preferred embodiment, the compositions comprise hydrophilic surfactants.

The hydrophilic surfactants are preferably selected from the group consisting of the alkylglucosides, acyl lactylates, betaines and coconut amphoacetates.

The alkylglucosides are themselves advantageously selected from the group consisting of the alkylglucosides which are distinguished by the structural formula

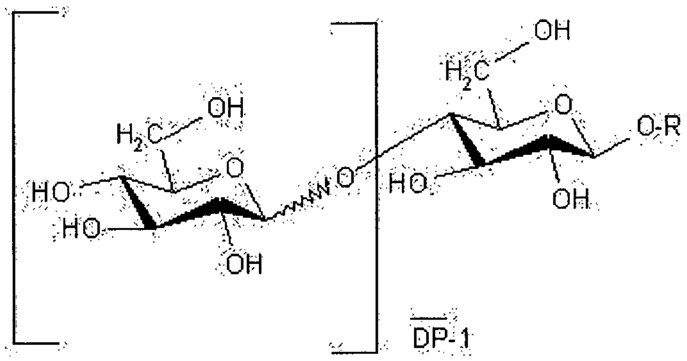

where

R is a branched or unbranched alkyl radical having from 4 to 24 carbon atoms, and $\overline{DP}$ denotes a mean degree of glucosylation of up to 2.

The value $\overline{DP}$ represents the degree of glucosidation of the alkylglucosides used is defined as

$$\overline{DP} = \frac{p_1}{100} \cdot 1 + \frac{p_2}{100} \cdot 2 + \frac{p_3}{100} \cdot 3 + \ldots = \Sigma \frac{p_i}{100} \cdot i$$

in which $p_1$, $p_2$, $p_3$ ... pi represent the proportion of mono-, di-, tri- ... i-fold glucosylated products in per cent by weight.

Products which are advantageous are those having degrees of glucosylation of 1-2, particularly advantageously of from 1.1 to 1.5, very particularly advantageously of 1.2-1.4, in particular of 1.3.

The value DP takes into account the fact that alkylglucosides are generally, as a consequence of their preparation, in the form of mixtures of mono- and oligoglucosides. A relatively high content of monoglucosides, typically in the order of 40-70% by weight, is advantageous.

Alkylglycosides which are particularly advantageously used are selected from the group consisting of octyl glucopyranoside, nonyl glucopyranoside, decyl glucopyranoside, undecyl glucopyranoside, dodecyl glucopyranoside, tetradecyl glucopyranoside and hexadecyl glucopyranoside.

It is likewise advantageous to employ natural or synthetic raw materials and assistants or mixtures which are distinguished by an effective content of the active ingredients used, for example Plantaren® 1200 (Henkel KGaA), Oramix® NS 10 (Seppic).

The acyllactylates are themselves advantageously selected from the group consisting of the substances which are distinguished by the structural formula

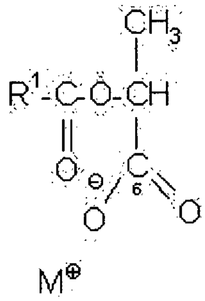

where

$R^1$ is a branched or unbranched alkyl radical having from 1 to 30 carbon atoms, and

$M^+$ is selected from the group consisting of the alkali metal ions and the group consisting of ammonium ions which are substituted by one or more alkyl and/or one or more hydroxyalkyl radicals, or corresponds to half an equivalent of an alkaline earth metal ion.

For example, sodium isostearyl lactylate, for example the product Pathionic® ISL from the American Ingredients Company, is advantageous.

The betaines are advantageously selected from the group consisting of the substances which are distinguished by the structural formulae

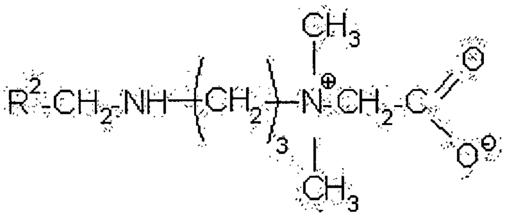

or

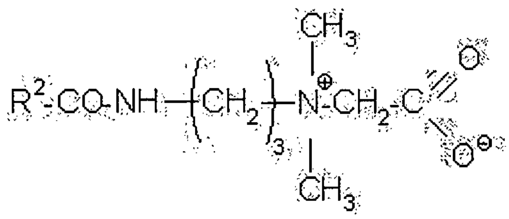

where
$R^2$ is a branched or unbranched alkyl radical having from 1 to 30 carbon atoms.

$R^2$ is particularly advantageously a branched or unbranched alkyl radical having from 6 to 12 carbon atoms.

For example, capramidopropylbetaine, for example the product Tego® Betain 810 from Th. Goldschmidt AG, is advantageous.

A coconut amphoacetate which is advantageous is, for example, sodium coconut amphoacetate, as available under the name Miranol® Ultra C32 from Miranol Chemical Corp.

The compositions are advantageously characterised in that the hydrophilic surfactant(s) is (are) present in concentrations of 0.01-20% by weight, preferably 0.05-10% by weight, particularly preferably 0.1-5% by weight, in each case based on the total weight of the composition.

For use, the cosmetic and dermatological compositions are applied in sufficient amount to the skin in the usual manner for cosmetics.

Cosmetic and dermatological compositions may exist in various forms. Thus, they may be, for example, a solution, a water-free composition, an emulsion or microemulsion of the water-in-oil (W/O) or oil-in-water (O/W) type, a multiple emulsion, for example of the water-in-oil-in-water (W/O/W) type, a gel, a solid stick, an ointment or an aerosol. It is also advantageous to administer ectoines in encapsulated form, for example in collagen matrices and other conventional encapsulation materials, for example as cellulose encapsulations, in gelatine, wax matrices or liposomally encapsulated. In particular, wax matrices, as described in DE-A 43 08 282, have proven favourable. Preference is given to emulsions. O/W emulsions are particularly preferred. Emulsions, W/O emulsions and O/W emulsions are obtainable in a conventional manner.

Emulsifiers that can be used are, for example, the known W/O and O/W emulsifiers. It is advantageous to use further conventional co-emulsifiers in the preferred O/W emulsions.

Co-emulsifiers which are advantageous are, for example, O/W emulsifiers, principally from the group consisting of the substances having HLB values of 11-16, very particularly advantageously having HLB values of 14.5-15.5, so long as the O/W emulsifiers have saturated radicals R and R'. If the O/W emulsifiers have unsaturated radicals R and/or R' or in the case of isoalkyl derivatives, the preferred HLB value of such emulsifiers may also be lower or higher.

It is advantageous to select the fatty alcohol ethoxylates from the group consisting of ethoxylated stearyl alcohols, cetyl alcohols, cetylstearyl alcohols (cetearyl alcohols). Particular preference is given to the following: polyethylene glycol (13) stearyl ether (steareth-13), polyethylene glycol (14) stearyl ether (steareth-14), polyethylene glycol (15) stearyl ether (steareth-15), polyethylene glycol (16) stearyl ether (steareth-16), polyethylene glycol (17) stearyl ether (steareth-17), polyethylene glycol (18) stearyl ether (steareth-18), polyethylene glycol (19) stearyl ether (steareth-19), polyethylene glycol (20) stearyl ether (steareth-20), polyethylene glycol (12) isostearyl ether (isosteareth-12), polyethylene glycol (13) isostearyl ether (isosteareth-13), polyethylene glycol (14) isostearyl ether (isosteareth-14), polyethylene glycol (15) isostearyl ether (isosteareth-15), polyethylene glycol (16) isostearyl ether (isosteareth-16), polyethylene glycol (17) isostearyl ether (isosteareth-17), polyethylene glycol (18) isostearyl ether (isosteareth-18), polyethylene glycol (19) isostearyl ether (isosteareth-19), polyethylene glycol (20) isostearyl ether (isosteareth-20), polyethylene glycol (13) cetyl ether (ceteth-13), polyethylene glycol (14) cetyl ether (ceteth-14), polyethylene glycol (15) cetyl ether (ceteth-15), polyethylene glycol (16) cetyl ether (ceteth-16), polyethylene glycol (17) cetyl ether (ceteth-17), polyethylene glycol (18) cetyl ether (ceteth-18), polyethylene glycol (19) cetyl ether (ceteth-19), polyethylene glycol (20) cetyl ether (ceteth-20), polyethylene glycol (13) isocetyl ether (isoceteth-13), polyethylene glycol (14) isocetyl ether (isoceteth-14), polyethylene glycol (15) isocetyl ether (isoceteth-15), polyethylene glycol (16) isocetyl ether (isoceteth-16), polyethylene glycol (17) isocetyl ether (isoceteth-17), polyethylene glycol (18) isocetyl ether (isoceteth-18), polyethylene glycol (19) isocetyl ether (isoceteth-19), polyethylene glycol (20) isocetyl ether (isoceteth-20), polyethylene glycol (12) oleyl ether (oleth-12), polyethylene glycol (13) oleyl ether (oleth-13), polyethylene glycol (14) oleyl ether (oleth-14), polyethylene glycol (15) oleyl ether (oleth-15), polyethylene glycol (12) lauryl ether (laureth-12), polyethylene glycol (12) isolauryl ether (isolaureth-12), polyethylene glycol (13) cetylstearyl ether (ceteareth-13), polyethylene glycol (14) cetylstearyl ether (ceteareth-14), polyethylene glycol (15) cetylstearyl ether (ceteareth-15), polyethylene glycol (16) cetylstearyl ether (ceteareth-16), polyethylene glycol (17) cetylstearyl ether (ceteareth-17), polyethylene glycol (18) cetylstearyl ether (ceteareth-18), polyethylene glycol (19) cetylstearyl ether (ceteareth-19), polyethylene glycol (20) cetylstearyl ether (ceteareth-20).

It is furthermore advantageous to select the fatty acid ethoxylates from the following group:

polyethylene glycol (20) stearate, polyethylene glycol (21) stearate, polyethylene glycol (22) stearate, polyethylene glycol (23) stearate, polyethylene glycol (24) stearate, polyethylene glycol (25) stearate, polyethylene glycol (12) isostearate, polyethylene glycol (13) isostearate, polyethylene glycol (14) isostearate, polyethylene glycol (15) isostearate, polyethylene glycol (16) isostearate, polyethylene glycol (17) isostearate, polyethylene glycol (18) isostearate, polyethylene glycol (19) isostearate, polyethylene glycol (20) isostearate, polyethylene glycol (21) isostearate, polyethylene glycol (22) isostearate, polyethylene glycol (23) isostearate, polyethylene glycol (24) isostearate, polyethylene glycol (25) isostearate, polyethylene glycol (12) oleate, polyethylene glycol (13) oleate, polyethylene glycol (14) oleate,

polyethylene glycol (15) oleate, polyethylene glycol (16) oleate, polyethylene glycol (17) oleate, polyethylene glycol (18) oleate, polyethylene glycol (19) oleate, polyethylene glycol (20) oleate.

An ethoxylated alkyl ether carboxylic acid or salt thereof which can advantageously be used is sodium laureth-11 carboxylate. An alkyl ether sulfate which can advantageously be used is sodium laureth-14 sulfate. An ethoxylated cholesterol derivative which can advantageously be used is polyethylene glycol (30) cholesteryl ether. Polyethylene glycol (25) soyasterol has also proven successful. Ethoxylated triglycerides which can advantageously be used are the polyethylene glycol (60) evening primrose glycerides.

It is furthermore advantageous to select the polyethylene glycol glycerol fatty acid esters from the group consisting of polyethylene glycol (20) glyceryl laurate, polyethylene glycol (21) glyceryl laurate, polyethylene glycol (22) glyceryl laurate, polyethylene glycol (23) glyceryl laurate, polyethylene glycol (6) glyceryl caprate/caprinate, polyethylene glycol (20) glyceryl oleate, polyethylene glycol (20) glyceryl isostearate, polyethylene glycol (18) glyceryl oleate/cocoate.

It is likewise favourable to select the sorbitan esters from the group consisting of polyethylene glycol (20) sorbitan monolaurate, polyethylene glycol (20) sorbitan monostearate, polyethylene glycol (20) sorbitan monoisostearate, polyethylene glycol (20) sorbitan monopalmitate, polyethylene glycol (20) sorbitan monooleate.

Optional W/O emulsifiers, but ones which may nevertheless be advantageous can be the following:

fatty alcohols having from 8 to 30 carbon atoms, monoglycerol esters of saturated and/or unsaturated, branched and/or unbranched alkanecarboxylic acids having a chain length of from 8 to 24 carbon atoms, in particular 12-18 carbon atoms, diglycerol esters of saturated and/or unsaturated, branched and/or unbranched alkanecarboxylic acids having a chain length of from 8 to 24 carbon atoms, in particular 12-18 carbon atoms, monoglycerol ethers of saturated and/or unsaturated, branched and/or unbranched alcohols having a chain length of from 8 to 24 carbon atoms, in particular 12-18 carbon atoms,

diglycerol ethers of saturated and/or unsaturated, branched and/or unbranched alcohols having a chain length of from 8 to 24 carbon atoms, in particular 12-18 carbon atoms, propylene glycol esters of saturated and/or unsaturated,

branched and/or unbranched alkanecarboxylic acids having a chain length of from 8 to 24 carbon atoms, in particular 12-18 carbon atoms, and sorbitan esters of saturated and/or unsaturated, branched and/or unbranched alkanecarboxylic acids having a chain length of from 8 to 24 carbon atoms, in particular 12-18 carbon atoms.

Particularly advantageous W/O emulsifiers are glyceryl monostearate, glyceryl monoisostearate, glyceryl monomyristate, glyceryl monooleate, diglyceryl monostearate, diglyceryl monoisostearate, propylene glycol monostearate, propylene glycol monoisostearate, propylene glycol monocaprylate, propylene glycol monolaurate, sorbitan monoisostearate, sorbitan monolaurate, sorbitan monocaprylate, sorbitan monoisooleate, sucrose distearate, cetyl alcohol, stearyl alcohol, arachidyl alcohol, behenyl alcohol, isobehenyl alcohol, selachyl alcohol, chimyl alcohol, polyethylene glycol (2) stearyl ether (steareth-2), glyceryl monolaurate, glyceryl monocaprinate and glyceryl monocaprylate.

Preferred compositions are particularly suitable for protecting human skin against ageing processes and against oxidative stress, i.e. against damage by free radicals, as are produced, for example, by sunlight, heat or other influences. In this connection, they are in the various administration forms usually used for this application. For example, they may, in particular, be in the form of a lotion or emulsion, such as in the form of a cream or milk (O/W, W/O, O/W/O, W/O/W), in the form of oily-alcoholic, oily-aqueous or aqueous-alcoholic gels or solutions, in the form of solid sticks or may be formulated as an aerosol.

The composition may comprise cosmetic adjuvants which are usually used in this type of composition, such as, for example, thickeners, softeners, moisturisers, surfactants, emulsifiers, preservatives, antifoams, perfumes, waxes, lanolin, propellants, dyes and/or pigments which colour the composition itself or the skin, and other ingredients usually used in cosmetics.

The dispersant or solubiliser used can be an oil, wax or other fatty substance, a lower monoalcohol or lower polyol or mixtures thereof. Particularly preferred monoalcohols or polyols include ethanol, isopropanol, propylene glycol, glycerol and sorbitol.

Preferred is an emulsion in the form of a protective cream or milk which, apart from the Compound comprises, for example, fatty alcohols, fatty acids, fatty acid esters, in particular triglycerides of fatty acids, lanolin, natural and synthetic oils or waxes and emulsifiers in the presence of water.

Further preferred are oily lotions based on natural or synthetic oils and waxes, lanolin, fatty acid esters, in particular triglycerides of fatty acids, or oily-alcoholic lotions based on a lower alcohol, such as ethanol, or a glycerol, such as propylene glycol, and/or a polyol, such as glycerol, and oils, waxes and fatty acid esters, such as triglycerides of fatty acids.

The composition may also be in the form of an alcoholic gel which comprises one or more lower alcohols or polyols, such as ethanol, propylene glycol or glycerol, and a thickener, such as siliceous earth. The oily-alcoholic gels also comprise natural or synthetic oil or wax.

The solid sticks consist of natural or synthetic waxes and oils, fatty alcohols, fatty acids, fatty acid esters, lanolin and other fatty substances.

If a composition is formulated as an aerosol, the customary propellants, such as alkanes, fluoroalkanes and chlorofluoroalkanes, are generally used.

A process for the preparation of a composition is characterised in that the Compound is mixed with a cosmetically or dermatologically or food-suitable carrier, and to the use of the Compound for the preparation of a composition. The compositions can be prepared here with the aid of techniques which are well known to the person skilled in the art.

The mixing can result in dissolution, emulsification or dispersal of the Compound.

The positive effects of the Compound give rise to their particular suitability for use in cosmetic compositions.

The properties of the Compound should likewise be regarded as positive for use in foods or as food supplements or as functional foods. The further explanations given for foods also apply correspondingly to food supplements and functional foods.

The foods which can be enriched with the Compound include all materials which are suitable for consumption by animals or consumption by humans, for example vitamins and provitamins thereof, fats, minerals or amino acids. (The foods may be solid, but also liquid, i.e. in the form of a beverage).

Foods which can be enriched with the Compound are, for example, also foods which originate from a single natural source, such as, for example, sugar, unsweetened juice, squash or puree of a single plant species, such as, for example, unsweetened apple juice (for example also a mixture of different types of apple juice), grapefruit juice, orange juice, apple compote, apricot squash, tomato juice, tomato sauce, tomato puree, etc. Further examples of foods which can be enriched with the Compound are corn or cereals from a single plant species and materials produced from plant species of this type, such as, for example, cereal syrup, rye flour, wheat flour or oat bran. Mixtures of foods of this type are also suitable for being enriched with the Compound are, for example, multivitamin preparations, mineral mixtures or sweetened juice. As further examples of foods which can be enriched with the Compound, mention may be made of food compositions, for example prepared cereals, biscuits, mixed drinks, foods prepared especially for children, such as yoghurt, diet foods, low-calorie foods or animal feeds.

The foods which can be enriched with the Compound thus include all edible combinations of carbohydrates, lipids, proteins, inorganic elements, trace elements, vitamins, water or active metabolites of plants and animals.

The foods which can be enriched with the Compound are preferably administered orally, for example in the form of meals, pills, tablets, capsules, powders, syrup, solutions or suspensions.

The foods enriched with the Compound can be prepared with the aid of techniques which are well known to the person skilled in the art.

The invention is explained in greater detail below by means of examples. The invention can be carried out throughout the range claimed and is not restricted to the examples given here.

Moreover, the following examples are given in order to assist the skilled artisan to better understand the present invention by way of exemplification.

Experimental

1. in vitro biological activities

Analysis of the activation of PPARα and PPARγ is based on the transfection of a DNA allowing the expression of a reporter gene (the gene of luciferase) under the control of the PPARs, either endogenous in the case of PPARγ or cotransfected in the case of PPARα. The reporter plasmid J3TkLuc comprises three copies of the response element for PPARs of the human apo A-II gene (Staels, B et al. (1995), J. Clin. Invest., 95, 705-712) which are cloned upstream of the promoter of the thymidine kinase gene of the herpes simplex virus in the plasmid pGL3. This reporter gene was obtained by subcloning, in the plasmid pGL3, the plasmid J3TkCAT described above (Fajas, L et al. (1997), J. Biol. Chem., 272, 18779-18789). The cells used are green monkey CV1 cells transformed by the SV40 virus, which express PPARγ (Forman, B. et al. (1995), Cell, 83, 803-812), and human SK-Hep1 cells, which do not express PPARs. These cells were inoculated at a density of 20,000 cells per well (96-well plates) and transfected with 150 ng of reporter DNA complexed with a mixture of lipids. In the case of the SK-Hep1 cells, an expression vector for PPARα, described by Sher, T. et al. (1993), Biochemistry, 32, 5598-5604, is cotransfected. After 5 hours, the cells are washed twice and incubated for 36 hours in the presence of the test compound in a fresh culture medium comprising 10% foetal calf serum. At the end of incubation, the cells are lysed and the luciferase activity is measured. This activity is expressed relative to the control value (data shown in Tables 1 and 2 )

**Table 1:** PPAR-alpha, mean of the two experiments (EXP 2050/C00CG1/050 and EXP 2137/C02CG1/140), performed in triplicate

| | Fold Activation PPAR alpha | | | | | | |
|---|---|---|---|---|---|---|---|
| | 100μm | 50μm | 30μm | 10μm | 3μm | 1μm | 0.3μm |
| EXP 2050/C00CG1/050 | 4.4 | 4.5 | 5.7 | 1.0 | 0.7 | 0.8 | 0.5 |
| | 2.9 | 3.0 | 2.8 | 0.9 | 0.6 | 0.6 | 0.7 |
| | 5.3 | 6.6 | 4.0 | 1.1 | 0.7 | 0.7 | 0.7 |
| EXP 2173/C02CG1/140 | 10.7 | 13.8 | 5.7 | 0.9 | 1.0 | 0.4 | 0.8 |
| | 7.8 | 12.0 | 5.5 | 2.1 | 1.1 | 1.0 | 0.6 |
| | 10.4 | 7.9 | 5.4 | 1.4 | 0.8 | 0.4 | 0.8 |
| MEAN | 6.9 | 8,0 | 4.9 | 1.2 | 0.8 | 0.7 | 1.4 |
| SEM | 1.3 | 1.7 | 0.5 | 0.2 | 0.1 | 0.1 | 0.8 |

**Table 2:** PPAR-gamma, mean of the two experiments (EXP 2050/C00CG1/050 and EXP 2137/C02CG1/140), performed in triplicate

| | Fold Activation PPAR gamma | | | | | | |
|---|---|---|---|---|---|---|---|
| | 100μm | 50μm | 30μm | 10μm | 3μm | 1μm | 0.3μm |
| EXP 2050/C00CG1/050 | 14.7 | 15.6 | 17.8 | 5.5 | 2.1 | 0.9 | 1.0 |
| | 16.3 | 14.5 | 13.4 | 5.9 | 1.2 | 0.8 | 0.8 |

(continued)

| | Fold Activation PPAR gamma | | | | | | |
|---|---|---|---|---|---|---|---|
| | 100μm | 50μm | 30μm | 10μm | 3μm | 1μm | 0.3μm |
| | 18.4 | 29.7 | 22.9 | 7.8 | 1.6 | 1.2 | 0.9 |
| EXP 2173/C02CG1/140 | 15.1 | 11.5 | 11.1 | 5.2 | 1.2 | 0.9 | 0.5 |
| | 17.5 | 24.4 | 9.5 | 4.5 | 1.3 | 1.0 | 1.2 |
| | 9.1 | 9.7 | 11.5 | 2.9 | 1.3 | 1.3 | 1.0 |
| MEAN | 15.2 | 17.6 | 14.4 | 5.2 | 1.5 | 1.0 | 0.9 |
| SEM | 1.3 | 3.2 | 2.1 | 0.7 | 0.1 | 0.1 | 0.1 |

Inhibition of Melanin synthesis in B16-V melanoma cells - Whitening Activity

B16-V melanoma cells were incubated for 72 h with assay medium containing different amounts of test substances. The concentration depends on the results of the MTT-Assay. Only concentrations without effect on viability (after 24 h incubation) were used for further melanin evaluation. Every 24 hours medium was changed and replaced by fresh assay medium containing the compounds and alpha-MSH (stimulation of melanin synthesis). The cell number was determined and the cells got lysed using 1 M NaOH. Afterwards the amount of melanin was measured at 405 nm with a microplatereader (Safire2TM, Tecan). Different concentrations of synthetic melanin were used to produce a calibration curve. Melanin content was expressed as pg/cell.

The test results are shown in Figure 1. As apparent from this Compound shows a superb melanin inhibiting activity. Furthermore the substance is significantly better than kojic acid.

2. cDNA-Microarray studies

One of the advantages of cDNA microarray is the possibility to observe the expression pattern of the whole genes and compare with different conditions. This is the first study to determine the gene expression profiles of Compound-treated human skin. For the analysis of deregulated gene expression induced by substance Compound, treated and control samples from a human skin model were analyzed with Affymetrix oligonucleotide microarrays. Skin has been among the first organs analysed using DNA-Microarrays. In addition, epidermal keratinocytes have been the target of many studies because they respond to a rich variety of inflammatory and immunmodulating cytokines, hormones, vitamins, ultraviolet (UV) light, toxins and physical injury. Altered gene expression after applying substance Compound was analysed with an Affymetrix system.

For gene expression studies skin equivalents were treated with 50μL of 2 relevant substance Compound with a concentration of c=50μM in PBS-buffer/DMSO. Buffer-treated skin equivalents served as controls.

Profiling of gene expression - Input genes

A ratio (log2) value of zero indicates no regulation, whereas positive (log2) values indicate an upregulation and negative (log2) values a downregulation of the relevant gene.

The experiment contains **759** genes with the specified threshold (Appendix A)

Data Analysis:

GeneGO's Metacore (Pathways and Maps) Software.

Results at first glance, several examples:

- The cDNA microarray analysis revealed that Compound deregulated relevant genes in the about 110 cellular and molecular processes whose content is defined and annotated by GeneGo. Each process represents a pre-set network of protein interactions characteristic for the process.

The investigation revealed that 14 of top 100 upregulated genes have at least one strong (high scored) site of PPAR.

Example 1: SCARB/ATP Binding Cassette/INSIG2A/LDR

One Interesting example is the upregulation of SCARB (Scavenger receptor class B, Probable receptor for HDL), INSIG2 (Insulin induced gene 2), ATP binding cassette and VLDLR genes, which Facilitate the flux of free and esterified cholesterol between the cell surface and extracellular donors and acceptors and regulate the cholesterol Homeostasis. PPAR-gamma is known to be the major transcription factor in this process (Christina Guggenberger, Dissertation 2007, institut für Experimentelle Genetik GSF-Forschungszentrum für Umwelt und Gesundheit, Neuherberg).

Example 2: PPAR/RXR

(not according to the invention as claimed)

Another interesting finding is that the Compound induces the upregulation of RXRA (retinoid X receptors A). The RAR/RXR transcription factor complex is activated by ligand binding, its natural ligand being all-trans retinoic acid (all-trans RA). The retinoid acid receptors (RAR) and peroxisome proliferator-activated receptors (PPAR) have been implicated in the regulation of inflammatory reactions (Sabien van Neerven et al, PPAR Research, vol 2007, Article ID 29275, 14 pages). Fenofibrate (a PPAR-alpha agonist) inhibited NF-kappaB DNA binding activity, suggesting a mechanism by which PPAR-alpha agonists may regulate the expression of genes encoding these pro-inflammatory molecules. Retinoid X receptors (RXRs) physically interact with PPAR-alpha receptors, and the resulting heterodimers regulate the expression of PPAR-responsive genes. Interestingly, a combination of 9-cis RA and the PPAR-alpha agonists fenofibrate or gemfibrozil cooperatively inhibited TNF-alpha, IL-1 beta, IL-6, and MCP-1 production by these cells. Collectively, these results raise the possibility that PPAR-alpha and RXR agonists might be effective in the treatment of MS (multiple sclerosis), where activated astrocytes are believed to contribute to disease pathology (J. Xu, et al., Journal of Neuroimmunology, vol. 176, no. 1-2, pp. 95-105, 2006).

Both receptor families contain ligand-activated transcription factors which form heterodimers with retinoid X receptors (RXR). Retinoid receptors play essential roles in the control of cell growth, differentiation and apoptosis. RXR is a combinatorial partner in the nuclear receptor family that forms homo- or heterodimers with a variety of hormone and orphan receptors, including retinoic acid receptor (RAR), Vitamin D receptor (VDR), thyroid hormone receptor (TR), peroxisome proliferators-activated receptor (PPAR), COUP-TF, and other receptors (Mangelsdorf, D. J.Cell, 83: 841-850, 1995).

RXRA is also abundantly expressed in the skin. Ablation of RXRα causes epidermal interfollicular hyperplasia, keratinocyte hyperproliferation, and aberrant terminal differentiation in skin (Li, M., et al., Development, 128: 675-688, 2001). RXRα-null F9 embryonal carcinoma cells are resistant to retinoid-mediated cellular differentiation, anti-proliferation, and apoptotis (Clifford, J., EMBO J., 15: 4142-4155, 1996).

Example 3: Inflammation/Psoriasis/Aging

(not according to the invention as claimed)

S100 calcium binding protein A7, S100 calcium binding protein A7A and S100 calcium binding protein A12 (S100A7, S100A7A and S100A12) are downregulated genes after treatment of skin cells with Compound.

S100A7A may be involved in epidermal differentiation and inflammation and might therefore be important for the pathogenesis of psoriasis and other diseases. The S100A7 protein, also known as psoriasin, has important functions as a mediator and regulator in skin differentiation and disease (psoriasis), in breast cancer, and as a chemotactic factor for inflammatory cells. (Kulski et al. Journal of Molecular Evolution (2003), 56(4), 397-406). Lener et al. investigate genes involved in the natural aging process of the human skin we applied cDNA microarray analysis of naturally aged human foreskin samples. They found that in total 105 genes change their expression over 1.7-fold during the aging process in the human skin. S100A7 has been described as upregulated gene, whereas GPC6 (Glypican 6) has been reported as dowregulated gene in old skin (T. Lener et al. / Experimental Gerontology 41 (2006) 387-397). Compound treated skin has opposite effect on GPC6 and S100A7 genes.

Caspase 14, P57/KIP2 are significantly upregulated in our experiment, comparable to the results of Stephan Hsu ((Stephen Hsu1 et al, Green Tea Polyphenol-Induced Epidermal Keratinocyte Differentiation is Associated with Coordinated Expression of p57/KIP2 and Caspase 14, JPET #76075), who has been reported, that EGCG (Epigallocatechin-3-gallate) induces caspase 14, a recently described putative regulator for keratinocyte terminal differentiation. They found that epidermal cells in psoriasis, a disease characterized by aberrant keratinization, exhibit reduced caspase

14 expression, particularly in the nuclear regions of the cells. Collectively, their data demonstrate that ECGC enabled normal human epidermal keratinocytes (NHEK) to accelerate terminal differentiation marked by a coordinated activation and expression of p57/KIP2 and caspase 14. Since caspase 14 is strictly associated with NHEK (normal human epidermal keratinocytes) terminal differentiation and skin barrier formation (Mikolajczyk et al, 2004), they propose that EGCG directs keratinocytes to enter a pathway leading to terminal differentiation and accelerated skin barrier formation. They suggested further that the EGCG-induced differentiation requires activation of both p57/KIP2 and caspase 14. The mRNA levels of p57/KIP2 and caspase 14 in NHEK were elevated by EGCG treatment.

Also, Cytokine IL-6, TNF-alpha induced proteins and Chemokines and their receptors IL-8, CCL8, CCL2, CXCL2, CXCL12, CXCL3 and CXCL5 and Intercellular adhesion molecule-1 (ICAM-1), which are known to be a major factor in modulating and directing inflammatory responses in the skin (Yasufumi Moromizato et al., Am J Pathol. 2000 October; 157(4): 1277-1281). Recently it has been reported that cell adhesion moldecules, including ICAM-1, are expressed on synovial fibroblasts in rheumatoid arthritis (RA) (Halle LP et al, Arthritis Rheum 1989; 32:22-29) are significantly down-regulated in our experiment. Intercellular adhesion molecule-1 (ICAM-1) is regularly expressed or inducible on all major cutaneous cell populations including Langerhans cells, keratinocytes, endothelial cells and dermal fibroblasts. ICAM-1 is induced in the skin under inflammatory conditions and plays an important role in the activation of T cells (R. Chatelain et al, Archives of dermatological research, vol. 290, number 9, Oct. 1998, 477-482).

Example 4: Stimulation of collagen and Improvment Elastin / Fibronectin / Laminin:

(not according to the invention as claimed)

Extracellular proteases are crucial regulators of cell function. The family of matrix metalloproteinases (MMPs) has classically been described in the context of extracellular matrix (ECM) remodelling, which occurs throughout life in diverse processes that range from tissue morphogenesis to wound healing. Recent evidence has implicated MMPs in the regulation of other functions, including survival, angiogenesis, inflammation and signalling. MMPs are part of a larger family of structurally related zinc-dependent metalloproteinases called metzincins. Other subfamilies of the metzincins are ADAMs, bacterial serralysins and the astacins.

MMPs are secreted from keratinocytes and fibroblasts and break down collagen and other proteins that comprise the dermal extracellular matrix. Imperfect repair of the dermal damage impairs the functional and structural integrity of the extracellular matrix. Repeated sun exposure causes accumulation of dermal damage that eventually results in characteristic wrinkling of photodamaged skin (Gary J. Fisher et al, ARCH DERMATOL/VOL 138, NOV 2002). In the skin, the primary role of MMP enzymes is to recycle skin matrix, particularly the structural proteins collagen and elastin. Reduced MMP activities degrade skin connective tissue and prevent loss of procollagen expression.

Type I collagen is the most abundant protein in skin, and type I and type III collagen fibrils provide strength and resiliency to skin. Photoaged skin contains an abundance of degraded, disorganized collagen fibrils and has a reduced production of type I and type III procollagen.

Decreasing the expression or activity of matrix metalloproteases, especially MMP-9, has an effect on the biological collagen catabolic process towards skin treatment of aging and psoriasis.

The down regulated MMP genes after treatment of skin cells with Compound are MMP9 (responsible for Gelatins, Collagens IV, V and XIV Aggrecan, Elastin), MMP14 (Collagens I, II and III, Laminin), MMP11 (Fibronectin). It is likely that downregulation of MMPs results in increase of collagen fibrils. In addition Fibronectin, Elastin and Laminin have been upregulated significantly. Laminin is thought to mediate the attachment, migration and organization of cells into tissues during embryonic development by interacting with other extracellular matrix components.

Also, the upregulated HAS2 gene (hyaluronan synthase 2) Plays a role in hyaluronan/hyaluronic acid (HA) synthesis.

Thus, Compound activates upregulation dermal fibroblast collagen production and downregulation of collagen degration.

Enrichment analysis of deregulated genes, automatically calculated using GeneGO

Enrichment analysis consists of matching gene IDs for the common, similar and unique sets of the uploaded files with gene IDs in functional ontologies in MetaCore. The ontologies include canonical pathway maps, GeneGo cellular processes, GO cellular processes and diseases categories. The degree of "relevance" to different categories for the uploaded datasets is defined by p-values, so that the lower p-value gets higher priority.

3. Cellular process:

Epidermis development (p-value: 3.1554e-08), cell differentiation (p-value: 2.2423e-08) and positive regulation

of follicle-stimulating hormone secretion (p-value 2.0701e-07) and hair follicle morphogenesis (p-value:2.274e-05), molting cycle process (p-value:8.996e-07), hair follicle development (p-value: 8.996e-07), hair cycle process (p-value: 8.996e-07), hair cycle (p-value: 1.005e-06), molting cycle (p-value:1.005e-06) are the outcoming cellular categories in our interest.

Example 1, Epidermis development:

(not according to the invention as claimed)

Epidermis development is the process whose specific outcome is the progression of the epidermis over time, from its formation to the mature structure. The epidermis is the outer epithelial layer of a plant or animal, it may be a single layer that produces an extracellular material (e.g. the cuticle of arthropods) or a complex stratified squamous epithelium, as in the case of many vertebrate species.

The GO/biological process categories are over-representative under the up- and down-regulated genes after treatment with the substance Compound. Following genes, which play an important role in Epidermis development, have been up- and downregulated (Table 3).

**Table 3:** Signal: ratio as log2 value of genes upregulated and downregulated by the Compound

| # | Gene Symbol | Protein | Protein name | Ratio as Log2 value |
|---|---|---|---|---|
| 49 | KRT34 | KRT34_HUMAN | Keratin, type I cuticular Ha4 | 1.695 |
| 10 | CASP14 | CASPE_HUMAN | Caspase 14 precursor | 1.44 |
| 58 | LCE2B | LCE2B_HUMAN | Late cornified envelope protein 2B | 1.326 |
| 24 | EMP1 | EMP1_HUMAN | Epithelial membrane protein 1 | 1.278 |
| 12 | CDSN | DCSN_HUMAN | Corneodesmosin precursor | 1.207 |
| 56 | LAMB3 | LAMB3_HUMAN | Laminin subunit beta-3 precursor | 1.168 |
| 62 | OVOL1 | OVOL1_HUMAN | Putative transcription factor Ovo-like 1 | 1.064 |
| 86 | UGCG | CEGT_HUMAN | Ceramide glucosyltransferase | 0.7977 |
| 55 | LAMA3 | LAMA3_HUMAN | Laminin subunit alpha-3 precursor | 0.6648 |
| 37 | KRT10 | K1C10_HUMAN | Keratin, type I cytoskeletal 10 | -0.6644 |
| 71 | S100a7 | S10A7_HUMAN | Protein S100-A7 | -0.6784 |
| 69 | PTHLH | PTHR_HUMAN | Parathyroid hormone-related protein | -0.7916 |
| 38 | KRT13 | K1C13_HUMAN | Keratin, type I cytoskeletal 13 | -0.8963 |
| 23 | ELF3 | ELF3_HUMAN | ETS-related transcription factor Elf-3 | -1.46 |

4. Formulation examples

Formulations for non-therapeutic compositions, cosmetic compositions and/or topical compositions, comprising the Compound are shown by way of example below. For some commercially available compounds the INCI names are given.

UV-Pearls™ OMC stands for the composition with the INCI: water (for EU: Aqua), Ethylhexyl Methoxycinnamate, Silica, PVP, Chlorphenesin, BHT; this composition is commercially available under the name Eusolex®UV Pearl™ OMC from Merck KGaA, Darmstadt.

The other UV Pearl products indicated in the tables are each of analogous composition with OMC replaced by the UV filter indicated.

Table 4a W/O-Emulsion (weight-%)

| | 1-1 | 1-2 | 1-3 | 1-4 | 1-5 | 1-9 |
|---|---|---|---|---|---|---|
| Titanium dioxide | | 2 | 5 | | | |
| Compound | 0,1 | 0,2 | 0,3 | 0,4 | 0,7 | 0,5 |

(continued)

| | 1-1 | 1-2 | 1-3 | 1-4 | 1-5 | 1-9 |
|---|---|---|---|---|---|---|
| Bisabolol | | 0,1 | | | | |
| Ectoine | 0,1 | | | | | |
| Tiliroside | | | 0,1 | | | |
| Allantoin | | | | | 0,1 | |
| Urea | | | | | | 2 |
| Zinc oxide | | | | | | 2 |
| UV-Pearl , OMC | 30 | 15 | 15 | 15 | 15 | 15 |
| Polyglyceryl-3-Dimerate | 3 | 3 | 3 | 3 | 3 | 3 |
| Cera Alba | 0,3 | 0,3 | 0,3 | 0,3 | 0,3 | 0,3 |
| Hydrogenated Castor Oil | 0,2 | 0,2 | 0,2 | 0,2 | 0,2 | 0,2 |
| Paraffinium Liquidum | 7 | 7 | 7 | 7 | 7 | 7 |
| Caprylic/Capric Triglyceride | 7 | 7 | 7 | 7 | 7 | 7 |
| Hexyl Laurate | 4 | 4 | 4 | 4 | 4 | 4 |
| PVP/Eicosene Copolymer | 2 | 2 | 2 | 2 | 2 | 2 |
| Propylene Glycol | 4 | 4 | 4 | 4 | 4 | 4 |
| Magnesium Sulfate | 0,6 | 0,6 | 0,6 | 0,6 | 0,6 | 0,6 |
| Tocopherol | 0,5 | 0,5 | 0,5 | 0,5 | 0,5 | 0,5 |
| Tocopheryl Acetate | 0,5 | 0,5 | 0,5 | 0,5 | 0,5 | 0,5 |
| Cyclomethicone | 0,5 | 0,5 | 0,5 | 0,5 | 0,5 | 0,5 |
| Propylparabene | 0,05 | 0,05 | 0,05 | 0,05 | 0,05 | 0,05 |
| Methylparabene | 0,15 | 0,15 | 0,15 | 0,15 | 0,15 | 0,15 |
| Water | ad 100 | ad 100 | ad 100 | ad 100 | ad 100 | ad 100 |

Table 4b

| | 1-11 | 1-12 | 1-13 | 1-14 | 1-15 | 1-16 | 1-17 | 1-18 |
|---|---|---|---|---|---|---|---|---|
| Titanium dioxide | 3 | | 2 | | 3 | | 2 | 5 |
| Benzylidene malonate polysiloxane | | 1 | 0,5 | | | | | |
| Methylene Bis-Benztriazolyl Tetramethylbutylphenol | 1 | 1 | 0,5 | | | | | |
| Compound | 0,1 | 0,2 | 0,3 | 0,5 | 0,7 | 0,2 | 0,4 | 0,2 |
| Polyglyceryl-3-Dimerate | 3 | 3 | 3 | 3 | | | | |
| Cera Alba | 0,3 | 0,3 | 0,3 | 0,3 | 2 | 2 | 2 | 2 |
| Hydrogenated Castor Oil | 0,2 | 0,2 | 0,2 | 0,2 | | | | |
| Paraffinium Liquidum | 7 | 7 | 7 | 7 | | | | |
| Caprylic/Capric Triglyceride | 7 | 7 | 7 | 7 | | | | |
| Hexyl Laurate | 4 | 4 | 4 | 4 | | | | |
| PVP/Eicosene Copolymer | 2 | 2 | 2 | 2 | | | | |
| Propylene Glycol | 4 | 4 | 4 | 4 | | | | |

(continued)

| | 1-11 | 1-12 | 1-13 | 1-14 | 1-15 | 1-16 | 1-17 | 1-18 |
|---|---|---|---|---|---|---|---|---|
| Magnesium Sulfate | 0,6 | 0,6 | 0,6 | 0,6 | | | | |
| Tocopherol | 0,5 | 0,5 | 0,5 | 0,5 | | | | |
| Tocopheryl Acetate | 0,5 | 0,5 | 0,5 | 0,5 | 1 | 1 | 1 | 1 |
| Cyclomethicone | 0,5 | 0,5 | 0,5 | 0,5 | | | | |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Dicocoyl Pentyerythrityl Citrate (and) Sorbitan Sesquioleate (and) Cera Alba (and) Aluminium Stearate | | | | | 6 | 6 | 6 | 6 |
| PEG-7 Hydrogenated Castor Oil | | | | | 1 | 1 | 1 | 1 |
| Zinc Stearate | | | | | 2 | 2 | 2 | 2 |
| Oleyl Erucate | | | | | 6 | 6 | 6 | 6 |
| Decyl Oleate | | | | | 6 | 6 | 6 | 6 |
| Dimethicone | | | | | 5 | 5 | 5 | 5 |
| Tromethamine | | | | | 1 | 1 | 1 | 1 |
| Glycerin | | | | | 5 | 5 | 5 | 5 |
| Allantoin | | | | | 0,2 | 0,2 | 0,2 | 0,2 |
| Water | ad 100 | ad 100 | ad 100 | ad 100 | ad 100 | ad 100 | ad 100 | ad 100 |

Table 5a: O/W-Emulsion, (weight-%)

| | 2-1 | 2-2 | 2-3 | 2-4 | 2-5 | 2-6 | 2-7 |
|---|---|---|---|---|---|---|---|
| Titanium dioxide | | 2 | 5 | | | | |
| Methylene Bis-Benztriazolyl Tetramethylbutylphenol | | | | | | 1 | 2 |
| Compound | 0,1 | 0,2 | 0,3 | 0,4 | 0,5 | 1 | 2 |
| 4-Methylbenzyliden Camphor | 2 | | 3 | | 4 | | 3 |
| BMDBM | 1 | 3 | | 3 | 3 | | 3 |
| Stearyl Alcohol (and) Steareth-7 (and) Steareth-10 | 3 | 3 | 3 | 3 | 3 | 3 | 3 |
| Glyceryl Stearate (and) Ceteth-20 | 3 | 3 | 3 | 3 | 3 | 3 | 3 |
| Glyceryl Stearate | 3 | 3 | 3 | 3 | 3 | 3 | 3 |
| Microwax | 1 | 1 | 1 | 1 | 1 | 1 | 1 |
| Cetearyl Octanoate | 11,5 | 11,5 | 11,5 | 11,5 | 11,5 | 11,5 | 11,5 |
| Caprylic/Capric Triglyceride | 6 | 6 | 6 | 6 | 6 | 6 | 6 |
| Oleyl Oleate | 6 | 6 | 6 | 6 | 6 | 6 | 6 |
| Propylene Glycol | 4 | 4 | 4 | 4 | 4 | 4 | 4 |
| Propylparabene | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 |

(continued)

| | 2-1 | 2-2 | 2-3 | 2-4 | 2-5 | 2-6 | 2-7 |
|---|---|---|---|---|---|---|---|
| Tromethamine | | | 1,8 | | | | |
| Water | ad 100 | ad 100 | ad 100 | ad 100 | ad 100 | ad 100 | ad 100 |

Table 5b

| | 2-11 | 2-12 | 2-13 | 2-14 | 2-15 | 2-16 | 2-17 | 2-18 |
|---|---|---|---|---|---|---|---|---|
| Titanium dioxide | 3 | | 2 | | | | 2 | 5 |
| Benzylidene malonate polysiloxane | | 1 | 0,5 | | | | | |
| Methylene Bis-Benztriazolyl Tetramethylbutylphenol | 1 | 1 | 0,5 | | | | | |
| Compound | 5 | 5 | 5 | 5 | 5 | 5 | 5 | 5 |
| Zinc oxide | | | 2 | | | | | |
| UV-Pearl, OMC | 15 | 15 | 15 | 30 | 30 | 30 | 15 | 15 |
| 4-Methylbenzyliden Camphor | | | | 3 | | | | |
| BMDBM | | | | 1 | | | | |
| Phenylbenzimidazole Sulfonic Acid | | | | | 4 | | | |
| Stearyl Alcohol (and) Steareth-7 (and) Steareth-10 | 3 | 3 | 3 | 3 | | | | |
| Glyceryl Stearate (and) Ceteth-20 | 3 | 3 | 3 | 3 | | | | |
| Glyceryl Stearate | 3 | 3 | 3 | 3 | | | | |
| Microwax | 1 | 1 | 1 | 1 | | | | |
| Cetearyl Octanoate | 11,5 | 11,5 | 11,5 | 11,5 | | | | |
| Caprylic/Capric Triglyceride | 6 | 6 | 6 | 6 | 14 | 14 | 14 | 14 |
| Oleyl Oleate | 6 | 6 | 6 | 6 | | | | |
| Propylene Glycol | 4 | 4 | 4 | 4 | | | | |
| Glyceryl Stearate SE | | | | | 6 | 6 | 6 | 6 |
| Stearic Acid | | | | | 2 | 2 | 2 | 2 |
| Persea Gratissima | | | | | 8 | 8 | 8 | 8 |
| Propylparabene | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 |
| Tromethamine | | | | | 1,8 | | | |
| Glycerin | | | | | 3 | 3 | 3 | 3 |
| Water | ad 100 | ad 100 | ad 100 | ad 100 | ad 100 | ad 100 | ad 100 | ad 100 |

Table 6: Gel, (weight-%)

| | 3-1 | 3-2 | 3-3 | 3-4 | 3-5 | 3-6 | 3-7 | 3-8 | 3-9 | 3-10 |
|---|---|---|---|---|---|---|---|---|---|---|
| | | | | | | | | | | |
| Titanium dioxide | | 2 | 5 | | | | | | | 3 |
| Compound | 0,05 | 0,1 | 0,3 | 0,2 | 0,5 | 0,6 | 0,7 | 0,8 | 0,9 | 1 |
| Benzylidene malonate polysiloxane | | | 1 | 1 | 2 | | | | 1 | 1 |
| Methylene Bis-Benztriazolyl Tetramethylbutylphenol | | 1 | | | | 1 | 2 | 1 | | |
| Zinc oxide | | | | 2 | | | | 5 | 2 | |
| UV-Pearl , Ethylhexyl Mehtoxycinnamat | 30 | 15 | 15 | 15 | 15 | 15 | 15 | 15 | 15 | 15 |
| 4-Methylbenzyliden Camphor | | | | | 2 | | | | | |
| Butylmethoxydibenzoylmethane | | 1 | | | | | | | | |
| Phenylbenzimidazole Sulfonic Acid | | | 4 | | | | | | | |
| Prunus Dulcis | 5 | 5 | 5 | 5 | 5 | 5 | 5 | 5 | 5 | 5 |
| Tocopheryl Acetate | 0,5 | 0,5 | 0,5 | 0,5 | 0,5 | 0,5 | 0,5 | 0,5 | 0,5 | 0,5 |
| Caprylic/Capric Triglyceride | 3 | 3 | 3 | 3 | 3 | 3 | 3 | 3 | 3 | 3 |
| Octyldodecanol | 2 | 2 | 2 | 2 | 2 | 2 | 2 | 2 | 2 | 2 |
| Decyl Oleate | 2 | 2 | 2 | 2 | 2 | 2 | 2 | 2 | 2 | 2 |
| PEG-8 (and) Tocopherol (and) Ascorbyl Palmitate (and) Ascorbic Acid (and) Citric Acid | 0,05 | 0,05 | 0,05 | 0,05 | 0,05 | 0,05 | 0,05 | 0,05 | 0,05 | 0,05 |
| Sorbitol | 4 | 4 | 4 | 4 | 4 | 4 | 4 | 4 | 4 | 4 |
| Polyacrylamide (and) C13-14 Isoparaffin (and) Laureth-7 | 3 | 3 | 3 | 3 | 3 | 3 | 3 | 3 | 3 | 3 |
| Propylparabene | 0,05 | 0,05 | 0,05 | 0,05 | 0,05 | 0,05 | 0,05 | 0,05 | 0,05 | 0,05 |
| Methylparabene | 0,15 | 0,15 | 0,15 | 0,15 | 0,15 | 0,15 | 0,15 | 0,15 | 0,15 | 0,15 |
| Tromethamine | | | 1,8 | | | | | | | |
| Water | ad 100 | ad 100 | ad 100 | ad 100 | ad 100 | ad 100 | ad 100 | ad 100 | ad 100 | ad 100 |

Table 7a: O/W emulsions (weight-%)

| A | INCI / Chem.-Name | **4-1** | **4-2** | **4-3** | **4-4** | **4-5** | **4-6** |
|---|---|---|---|---|---|---|---|
| | ARACHIDYL ALCOHOL, BEHENYL ALCOHOL, ARACHIDYLGLUCOSIDE | 3,00 | 3,00 | 3,00 | 3,00 | 3,00 | 3,00 |
| | PHENYLETHYL BENZOATE | 10,00 | 10,00 | 10,00 | 10,00 | 10,00 | 10,00 |
| | ISOPROPYLPHTALIMIDE, BUTYLPHTALIDE | 2,00 | 2,00 | 2,00 | 2,00 | 2,00 | 2,00 |
| | ISOEICOSANE | 1,00 | 1,00 | 1,00 | 1,00 | 1,00 | 1,00 |
| | HYDROGENATED POLYDECENE | 1,00 | 1,00 | 1,00 | 1,00 | 1,00 | 1,00 |
| | Compound | 0,2 | 0,1 | 0,3 | 0,5 | 1 | 5 |

(continued)

| A | INCI / Chem.-Name | **4-1** | **4-2** | **4-3** | **4-4** | **4-5** | **4-6** |
|---|---|---|---|---|---|---|---|
| B | GLYCERIN, AQUA | 3,00 | 3,00 | 3,00 | 3,00 | 3,00 | 3,00 |
| | AQUA (WATER) | Ad 100 | Ad 100 | Ad 100 | Ad 100 | Ad 100 | Ad 100 |
| | | | | | | | |
| | | | | | | | |
| C | HYDROXYETHYL ACRYLATE/SODIUM ACRYLOYLDIMETHYLTAURATE COPOLYMER, SQUALANE, POLYSORBATE 60 | 2,00 | 2,00 | 2,00 | 2,00 | 2,00 | 2,00 |
| | | | | | | | |
| D | PROPYLENE GLYCOL, DIAZOLIDINYL UREA, METHYLPARABEN, PROPYLPARABEN | 0,75 | 0,75 | 0,75 | 0,75 | 0,75 | 0,75 |

Table 7b

| **INCI** | **4-7** | **4-8** | **4-9** | **4-10** | **4-11** | **4-12** | **4-13** | **4-14** | **4-15** | **4-16** |
|---|---|---|---|---|---|---|---|---|---|---|
| | | | | | | | | | | |
| POLYGLYCERYL-3 METHYLGLUCOSE DISTEARATE | 3,00 | 3,00 | 3,00 | 3,00 | 3,00 | 3,00 | 3,00 | 3,00 | 3,00 | 3,00 |
| STEARYL ALCOHOL | 3,00 | 3,00 | 3,00 | 3,00 | 3,00 | 3,00 | 3,00 | 3,00 | 3,00 | 3,00 |
| GLYCERYL STEARATE | 2,00 | 2,00 | 2,00 | 2,00 | 2,00 | 2,00 | 2,00 | 2,00 | 2,00 | 2,00 |
| DECYL COCOATE | 5,00 | 5,00 | 5,00 | 5,00 | 5,00 | 5,00 | 5,00 | 5,00 | 5,00 | 5,00 |
| CETEARYL ETHYLHEXANOATE | 5,00 | 5,00 | 5,00 | 5,00 | 5,00 | 5,00 | 5,00 | 5,00 | 5,00 | 5,00 |
| C12-15 ALKYL BENZOATE | 8,50 | 8,50 | 8,50 | 8,50 | 8,50 | 8,50 | 8,50 | 8,50 | 8,50 | 8,50 |
| XANTHAN GUM | 0,20 | 0,20 | 0,20 | 0,20 | 0,20 | 0,20 | 0,20 | 0,20 | 0,20 | 0,20 |
| PROPYLPARABEN | 0,05 | 0,05 | 0,05 | 0,05 | 0,05 | 0,05 | 0,05 | 0,05 | 0,05 | 0,05 |
| Compound | 0,25 | 0,5 | 0,3 | 0,1 | 0,2 | 1 | 2 | 5 | 0,35 | 0,05 |
| GLYCERIN | 6,00 | 6,00 | 6,00 | 6,00 | 6,00 | 6,00 | 6,00 | 6,00 | 6,00 | 6,00 |
| METHYLPARABEN | 0,15 | 0,15 | 0,15 | 0,15 | 0,15 | 0,15 | 0,15 | 0,15 | 0,15 | 0,15 |
| AQUA (WATER) | Ad 100 | Ad 100 | Ad 100 | Ad 100 | Ad 100 | Ad 100 | Ad 100 | Ad 100 | Ad 100 | Ad 100 |

Table 8: W/O emulsions, (weight-%)

| | **5-1** | **5-2** | **5-3** | **5-4** | **5-6** | **5-7** | **5-8** | **5-9** | **5-10** |
|---|---|---|---|---|---|---|---|---|---|
| POLYGLYCERYL-4 ISOSTEARATE | 1,00 | 1,00 | 1,00 | 1,00 | 1,00 | 1,00 | 1,00 | 1,00 | 1,00 |
| CETYL PEG/PPG-10/1 DIMETHICONE | 2,00 | 2,00 | 2,00 | 2,00 | 2,00 | 2,00 | 2,00 | 2,00 | 2,00 |

(continued)

| | 5-1 | 5-2 | 5-3 | 5-4 | 5-6 | 5-7 | 5-8 | 5-9 | 5-10 |
|---|---|---|---|---|---|---|---|---|---|
| CERESIN (MICROCRYSTALLINE WAX) | 1,50 | 1,50 | 1,50 | 1,50 | 1,50 | 1,50 | 1,50 | 1,50 | 1,50 |
| HYDROGENATED CASTOR OIL | 0,50 | 0,50 | 0,50 | 0,50 | 0,50 | 0,50 | 0,50 | 0,50 | 0,50 |
| DECYL OLEATE | 10,00 | 10,00 | 10,00 | 10,00 | 10,00 | 10,00 | 10,00 | 10,00 | 10,00 |
| CAPRYLIC/CAPRIC TRIGLYCERIDE | 10,00 | 10,00 | 10,00 | 10,00 | 10,00 | 10,00 | 10,00 | 10,00 | 10,00 |
| OCTYLDODECYL MYRISTATE | 5,00 | 5,00 | 5,00 | 5,00 | 5,00 | 5,00 | 5,00 | 5,00 | 5,00 |
| Compound | 0,1 | 0,25 | 0,4 | 0,2 | 0,3 | 0,5 | 0,6 | 0,7 | 1 |
| GLYCERIN | 2,00 | 2,00 | 2,00 | 2,00 | 2,00 | 2,00 | 2,00 | 2,00 | 2,00 |
| MAGNESIUM SULFATE | 1,00 | 1,00 | 1,00 | 1,00 | 1,00 | 1,00 | 1,00 | 1,00 | 1,00 |
| AQUA (WATER) | 66,25 | 66,25 | 66,25 | 66,25 | 66,25 | 66,25 | 66,25 | 66,25 | 66,25 |
| PROPYLENE GLYCOL, DIAZOLIDINYL UREA, METHYLPARABEN, PROPYLPARABEN | 0,50 | 0,50 | 0,50 | 0,50 | 0,50 | 0,50 | 0,50 | 0,50 | 0,50 |

Table 9: ointment (water-free)

| | 6-1 | 6-2 | 6-3 | 6-4 | 6-5 | 6-6 | 6-7 | 6-8 | 6-9 | 6-10 |
|---|---|---|---|---|---|---|---|---|---|---|
| MICROWAX | 16,00 | 16,00 | 16,00 | 16,00 | 16,00 | 16,00 | 16,00 | 16,00 | 16,00 | 16,00 |
| BUTYROSPERMUM PARKII (SHEA BUTTER) | 13,00 | 13,00 | 13,00 | 13,00 | 13,00 | 13,00 | 13,00 | 13,00 | 13,00 | 13,00 |
| C12-15 ALKYL BENZOATE | 28,00 | 28,00 | 28,00 | 28,00 | 28,00 | 28,00 | 28,00 | 28,00 | 28,00 | 28,00 |
| CAPRYLIC/CAPRIC TRIGLYCERIDE | 27,25 | 27,25 | 27,25 | 27,25 | 27,25 | 27,25 | 27,25 | 27,25 | 27,25 | 27,25 |
| DIBUTYL ADIPATE | 15,00 | 15,00 | 15,00 | 15,00 | 15,00 | 15,00 | 15,00 | 15,00 | 15,00 | 15,00 |
| Compound | 0,1 | 0,25 | 0,4 | 0,3 | 1 | 0,5 | 0,9 | 0,6 | 0,75 | 5 |

Table 10: Oil-Gel (water-free)

| INCI | 7-1 | 7-2 | 7-3 | 7-4 | 7-5 | 7-6 | 7-7 | 7-8 | 7-9 | 7-10 |
|---|---|---|---|---|---|---|---|---|---|---|
| | | | | | | | | | | |
| OCTOCRYLENE | 10.00 | 10.00 | 10.00 | 10.00 | 10.00 | 10.00 | 10.00 | 10.00 | 10.00 | 10.00 |
| BUTYL METHOXYDIBENZOYL-METHANE | 3.00 | 3.00 | 3.00 | 3.00 | 3.00 | 3.00 | 3.00 | 3.00 | 3.00 | 3.00 |
| TOCOPHERYL ACETATE | 1.00 | 1.00 | 1.00 | 1.00 | 1.00 | 1.00 | 1.00 | 1.00 | 1.00 | 1.00 |
| TOCOPHEROL | 0.50 | 0.50 | 0.50 | 0.50 | 0.50 | 0.50 | 0.50 | 0.50 | 0.50 | 0.50 |
| ASCORBYL PALMITATE | 0.20 | 0.20 | 0.20 | 0.20 | 0.20 | 0.20 | 0.20 | 0.20 | 0.20 | 0.20 |
| GLYCERYL STEARATE | 0.10 | 0.10 | 0.10 | 0.10 | 0.10 | 0.10 | 0.10 | 0.10 | 0.10 | 0.10 |
| DIETHYLHEXY L CARBONATE | 2.00 | 2.00 | 2.00 | 2.00 | 2.00 | 2.00 | 2.00 | 2.00 | 2.00 | 2.00 |
| DECYL OLEATE | 2.00 | 2.00 | 2.00 | 2.00 | 2.00 | 2.00 | 2.00 | 2.00 | 2.00 | 2.00 |
| TRIDECYL SALICYLATE | 4.00 | 4.00 | 4.00 | 4.00 | 4.00 | 4.00 | 4.00 | 4.00 | 4.00 | 4.00 |
| Compound | 0,1 | 0,25 | 0,4 | 0,3 | 0,5 | 1 | 0,7 | 0,6 | 0,8 | 1 |
| ECTOIN | 0.30 | 0.30 | 0.30 | 0.30 | 0.30 | 0.30 | 0.30 | 0.30 | 0.30 | 0.30 |
| CITRIC ACID | 0.10 | 0.10 | 0.10 | 0.10 | 0.10 | 0.10 | 0.10 | 0.10 | 0.10 | 0.10 |
| AQUA (WATER), CI 17200 | 0.30 | 0.30 | 0.30 | 0.30 | 0.30 | 0.30 | 0.30 | 0.30 | 0.30 | 0.30 |
| GLYCERIN | 1.00 | 1.00 | 1.00 | 1.00 | 1.00 | 1.00 | 1.00 | 1.00 | 1.00 | 1.00 |
| HYDROXY-PROPYLCELLULOSE | 2.00 | 2.00 | 2.00 | 2.00 | 2.00 | 2.00 | 2.00 | 2.00 | 2.00 | 2.00 |
| PROPYLENE GLYCOL | 3.00 | 3.00 | 3.00 | 3.00 | 3.00 | 3.00 | 3.00 | 3.00 | 3.00 | 3.00 |
| CYCLOHEXASILOXANE | 9.00 | 9.00 | 9.00 | 9.00 | 9.00 | 9.00 | 9.00 | 9.00 | 9.00 | 9.00 |
| ALCOHOL | Ad 100 | Ad 100 | Ad 100 | Ad 100 | Ad 100 | Ad 100 | Ad 100 | Ad 100 | Ad 100 | Ad 100 |

Table 11: Emulsion with 0.5 % Compound

| Phase | Trade Name | INCI Name | % w/w |
|---|---|---|---|
| A | Aqua<br>Germall II<br>Natrlquest E30<br>Glycerin<br>Akoline PG7<br>Akoline SL<br>Xanthan Gum | Aqua<br>Diazolidinyl Urea<br>Trisodiumethyllenediamine Disuccinate<br>Glycerin<br>Polyglyceryl-3 stearate<br>Sodium stearoyl lactylate<br>Xanthan Gum | 66.97<br>0.25<br>0.30<br>1.00<br>3.50<br>0.50<br>0.15 |
| B | Ceraphyl 230<br>Compound<br>Phenoxyethanol<br>Oxynex ST Liquid<br>Lanette 16<br>Stearic Acid<br>Tegin M | Diisopropyl Adipate<br><br>Phenoxyethanol<br>Diethylhexyl Syringylidene Malonate<br>Cetyl Alcohol<br>Stearic Acid<br>Glyceryl Syearate | 22.00<br>0.50<br>0.60<br>0.05<br>2.00<br>1.00<br>1.00 |
| C | Acido Lattico | Lactic Acid | 0.18 |
| | | | **100.00** |

pH = 4.36
Viscosity (25°C) Helipath T-C 2.5 rpm = 220,000 mPa.s
5 rpm = 110,000 mPa.s

Organoleptically stable after 3 months at 42.5 °C, 4°C and room temperature.

Table 12: Emulsion with 0.1 % Compound

| Phase | Trade Name | INCI Name | % w/w |
|---|---|---|---|
| A | Aqua<br>Germall II<br>Natrlquest E30<br>Glycerin<br>Akoline PG7<br>Akoline SL<br>Xanthan Gum | Aqua<br>Diazolidinyl Urea<br>Trisodiumethyllenediamine Disuccinate<br>Glycerin<br>Polyglyceryl-3 stearate<br>Sodium stearoyl lactylate<br>Xanthan Gum | 84.97<br>0.25<br>0.30<br>1.00<br>3.50<br>0.50<br>0.15 |
| B | Ceraphyl 230<br>Compound<br>Phenoxyethanol<br>Oxynex ST Liquid<br>Lanette 16<br>Stearic Acid<br>Tegin M | Diisopropyl Adipate<br><br>Phenoxyethanol<br>Diethylhexyl Syringylidene Malonate<br>Cetyl Alcohol<br>Stearic Acid<br>Glyceryl Syearate | 4.40<br>0.10<br>0.60<br>0.05<br>2.00<br>1.00<br>1.00 |
| C | Acido Lattico | Lactic Acid | 0.18 |
| | | | **100.00** |

pH = 4.15
Viscosità (25°C) Helipath T-C 2.5 rpm = 200,000 mPa.s
5 rpm = 100,000 mPa.s

The constituents of phases A and B are each mixed separately from one another, then phase B is added to phase A. Phase C is added to the mixture of phases A and B with stirring.

Organoleptically stable after 3 months at 42.5 °C, 4°C and room temperature.

Table 13: Lotion with 0.5% Compound

| Phase | Trade Name | INCI Name | % w/w |
|---|---|---|---|
| **A** | Aqua<br>Arlasolv DMI<br>Ethanol<br>Compound | Aqua<br>Dimethyl isosorbide<br>Ethanol | 33.50<br>33.00<br>33.00<br>0.50 |
| | | | **100.00** |

The constituents of phase A are each mixed together.
pH = 3.98
Viscosity (25°C) < 20 mPa.s

Organoleptically stable after 3 months at 42.5 °C, 4°C and room temperature.

Table 14: Lotion with 0.1% Compound

| Phase | Trade Name | INCI Name | % w/w |
|---|---|---|---|
| **A** | Aqua<br>Arlasolv DMI<br>Ethanol<br>Compound | Aqua<br>Dimethyl isosorbide<br>Ethanol | 33.50<br>33.00<br>33.00<br>0.10 |
| | | | **100.00** |

The constituents of phase A are each mixed together.
pH = 4.10
Viscosity (25°C) < 20 mPa.s

Organoleptically stable after 3 months at 42.5 °C, 4°C and room temperature.

6. In-vivo Studies:

Skin whitening activity was evaluated in a double blind clinical trial. As study medication a oil-in-water emulsion formulation containing 1.0% of the Compound, in comparison with the same formulation containing no active ingredient (placebo) was used.

The study was executed with subjects of skin type 3 or 4, aged from 18 to 50 years, whereby the active formulation was applied on one forearm twice a day and the placebo formulation on the other forearm for 4½ weeks. Assessment of whitening efficacy was performed on both forearms at day 0, 14 and 28 by visual assessment (examination of pigmentation differences), colorimetric measurements (using Chroma-Meter CR 300, *Minolta* resulting in L* values, expressing the changes in brightness, and ITA° values *Individual Typology Angle* expressing the melanin index) as well as digital photographs.

The data obtained by visual assessment are shown in Fig. 2, the data obtained in Chromametric assessement in Fig. 3 (L* values) and Fig. 4 (ITA° values). As is clearly indicated by such data the presence of the Compound in the formulation leads to an extraordinary skin whitening activity which is highly statistically significant.

**Appendix A**

| ID-No. | Gene Symbol | Protein | Protein name | Compound vs. Control, ratio Log(2) value |
|---|---|---|---|---|
| 3 | ABCG1 | ABCG1_HUMAN | ATP-binding cassette sub-family G member 1 | 1,297 |
| 101 | CASP14 | CASE_HUMAN | Caspase-14 precursor | 1,44 |

(continued)

| ID-No. | Gene Symbol | Protein | Protein name | Compound vs. Control, ratio Log(2) value |
|---|---|---|---|---|
| 104 | CCL2 | CCL2_HUMAN | C-C motif chemokine 2 precursor | -1,731 |
| 106 | CCL8 | CCL8_HUMAN | C-C motif chemokine 8 precursor | -2,505 |
| 110 | CDKN1C | CDN1C_HUMAN | Cyclin-dependent kinase inhibitor 1C | 0,8737 |
| 126 | CLDN17 | CLD17_HUMAN | Claudin-17 | 2,19 |
| 135 | COL4A3BP | C43BP_HUMAN | Collagen type IV alpha-3-binding protein | 1,233 |
| 146 | CXCL12 | SDF1_HUMAN | Stromal cell-derived factor 1 precursor | -0,8377 |
| 147 | CXCL2 | MIP2A_HUMAN | Macrophage inflammatory protein 2-alpha precursor | -1,267 |
| 148 | CXCL3 | MIP2B_HUMAN | Macrophage inflammatory protein 2-beta precursor | -0,723 |
| 149 | CXCL5 | CXCL5_HUMAN | C-X-C motif chemokine 5 precursor | -0,8263 |
| 117 | CERK | CERK1_HUMAN | Ceramide kinase | 0.729 |
| 185 | EGFR | EGFR_HUMAN | Epidermal growth factor receptor precursor | 0.9876 |
| 191 | ELF3 | ELF3_HUMAN | ETS-related transcription factor Elf-3 | -1,46 |
| 194 | ELN | ELN_HUMAN | Elastin precursor | 0,7751 |
| 196 | EMP1 | EMP1_HUMAN | Epithelial membrane protein 1 | 1.278 |
| 197 | EMR2 | EMR2_HUMAN | EGF-like module-containing mucin-like hormone receptor-like 2 precursor | 1.286 |
| 214 | FABP4 | FABPA HUMAN | Fatty acid-binding protein, adipocyte | 2.125 |
| 241 | FLNB | FLNB_HUMAN | Filamin-B | 0,6681 |
| 243 | FN1 | FINC_HUMAN | Fibronectin precursor | 1,364 |
| 233 | FGF11 | FGF11 HUMAN | Fibroblast growth factor 11 | 0.9322 |
| 263 | GPC4 | GPC4_HUMAN | Glypican-4 precursor | 0,7558 |
| 264 | GPC6 | GPC6_HUMAN | Glypican-6 precursor | 0,6711 |
| 267 | GPX7 | GPX7_HUMAN | Glutathione peroxidase 7 precursor | 0.8162 |
| 275 | HAS2 | HAS2_HUMAN | Hyaluronan synthase 2 | 0,8154 |
| 294 | ICAM1 | ICAM1_HUMAN | Intercellular adhesion molecule 1 precursor | -1,612 |
| 312 | IL6 | IL6_HUMAN | Interleukin-6 precursor | -1,09 |
| 313 | IL6ST | IL6RB_HUMAN | Interleukin-6 receptor subunit beta precursor | -1,628 |
| 314 | IL8 | IL8_HUMAN | Interleukin-8 precursor | -0,6883 |
| 323 | ITGB5 | ITB5_HUMAN | Integrin beta-5 precursor | 0.7305 |
| 324 | ITIH5 | ITIH5_80760 | inter-alpha (globulin) inhibitor H5 | 1.192 |
| 317 | INSIG2 | INSI2_HUMAN | Insulin-induced gene 2 protein | 0,6645 |
| 349 | LAMA3 | LAMA3_HUMAN | Laminin subunit alpha-3 precursor | 0,6648 |
| 350 | LAMB3 | LAMB3_HUMAN | Laminin subunit beta-3 precursor | 1,168 |

(continued)

| ID-No. | Gene Symbol | Protein | Protein name | Compound vs. Control, ratio Log(2) value |
|---|---|---|---|---|
| 351 | LAMC1 | LAMC1_HUMAN | Laminin subunit gamma-1 precursor | 0,7744 |
| 358 | LEP | LEP_HUMAN | Leptin precursor | 3.891 |
| 359 | LEPR | LEPR_HUMAN | Leptin receptor precursor | 0.7216 |
| 423 | MMP11 | MMP11_HUMAN | Stromelysin-3 precursor | -1,085 |
| 424 | MMP14 | MMP14_HUMAN | Matrix metalloproteinase-14 precursor | -0,6909 |
| 426 | MMP9 | MMP9_HUMAN | Matrix metalloproteinase-9 precursor | -0,8347 |
| 511 | PLOD2 | PLOD2 HUMAN | Procollagen-lysine,2-oxoglutarate 5-dioxygenase 2 precursor | 1.455 |
| 580 | RXRA | RXRA_HUMAN | Retinoic acid receptor RXR-alpha | 0,7793 |
| 582 | S100A12 | S10AC_HUMAN | Protein S100-A12 | -0,7202 |
| 583 | S100A7 | S10A7_HUMAN | Protein S100-A7 | -0,6784 |
| 584 | S100A7A | S1A7A_HUMAN | Protein S100-A7A | -2,659 |
| 587 | SCARB1 | SCRB1_HUMAN | Scavenger receptor class B member 1 | 0,8349 |
| 670 | TGFA | TGFA HUMAN | Protransforming growth factor alpha precursor [Contains: Transforming growth factor alpha | 0.8031 |
| 715 | UGCG | CEGT_HUMAN | Ceramide glucosyltransferase | 0.7977 |
| 726 | VLDLR | VLDLR_HUMAN | Very low-density lipoprotein receptor precursor | 0,9666 |

## Claims

1. Non-therapeutic use of 5-(7-methoxy-3,3-dimethyl-2,3-dihydro-1-benzoxepin-5-yl)-3-methyl-penta-2,4-dienoic acid and/or a salt or solvate thereof for skin lightening and/or skin whitening.

## Patentansprüche

1. Nichttherapeutische Verwendung von 5-(7-Methoxy-3,3-dimethyl-2,3-dihydro-1-benzoxepin-5-yl)-3-methyl-penta-2,4-diensäure und/oder eines Salzes oder Solvates davon zur Hautaufhellung und/oder Hautbleichung.

## Revendications

1. Utilisation non thérapeutique d'acide 5-(7-méthoxy-3,3-diméthyl-2,3-dihydro-1-benzoxépin-5-yl)-3-méthyl-penta-2,4-diénoïque et/ou d'un sel ou solvate de celui-ci, pour l'éclaircissement de la peau et/ou le blanchiment de la peau.

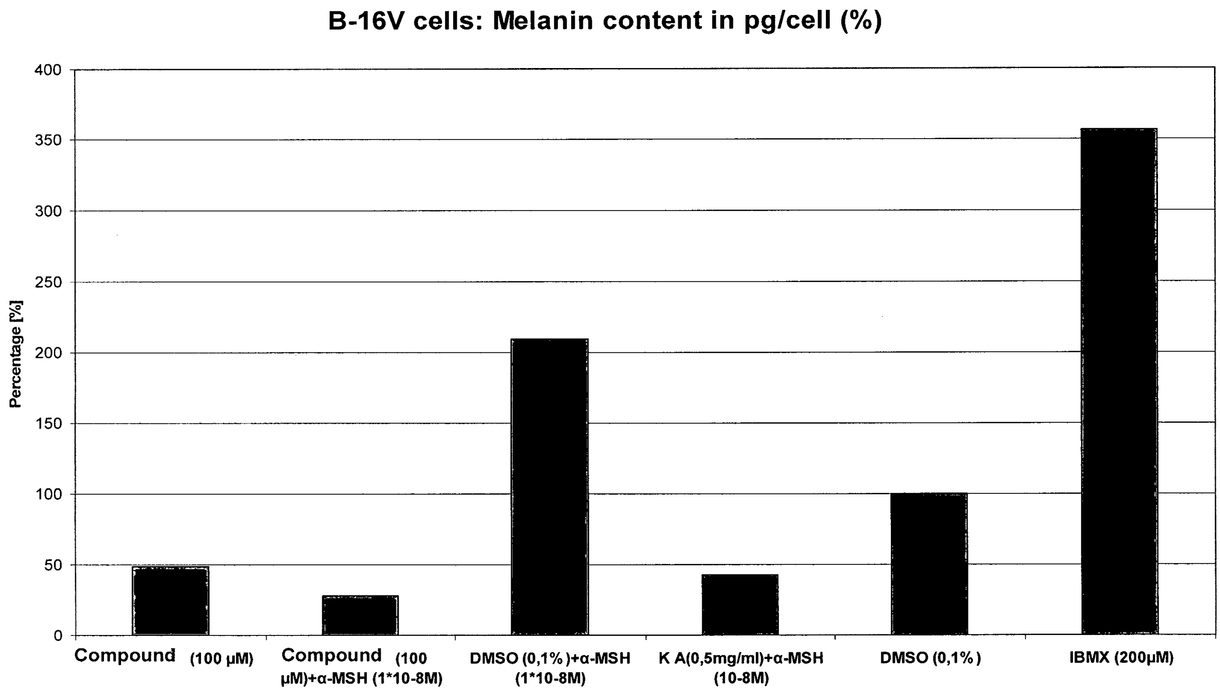


Fig. 1: B-16V cells: % of Melanin content in pg/cell. DMSO is the solvent as negative and IBMX (3-Isobutyl-1-methylxantine) is our positive control. Kojic acid (KA) is the known reference ingredient for skin whitening.

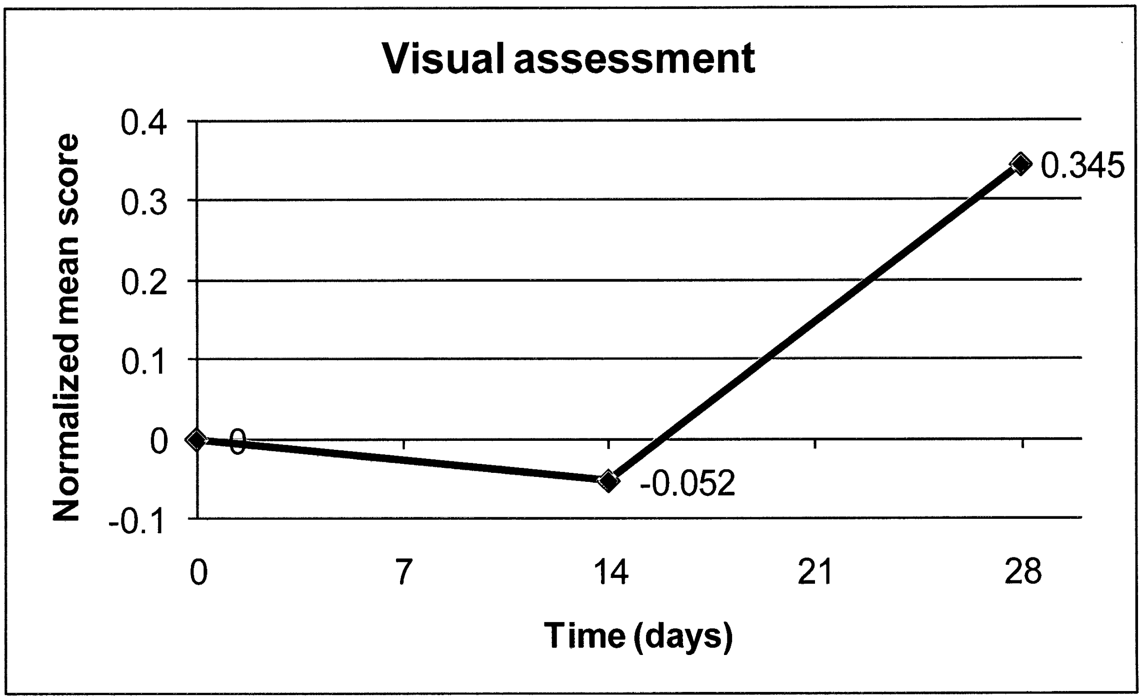


Fig. 2: The normalized mean score of the visual assessment averaged over the whole panel. A positive number indicates that the formulation containing the active was whiter than the placebo formulation. Only the value at Day 28 was statistically significantly different from Day 0 (p=0.022)

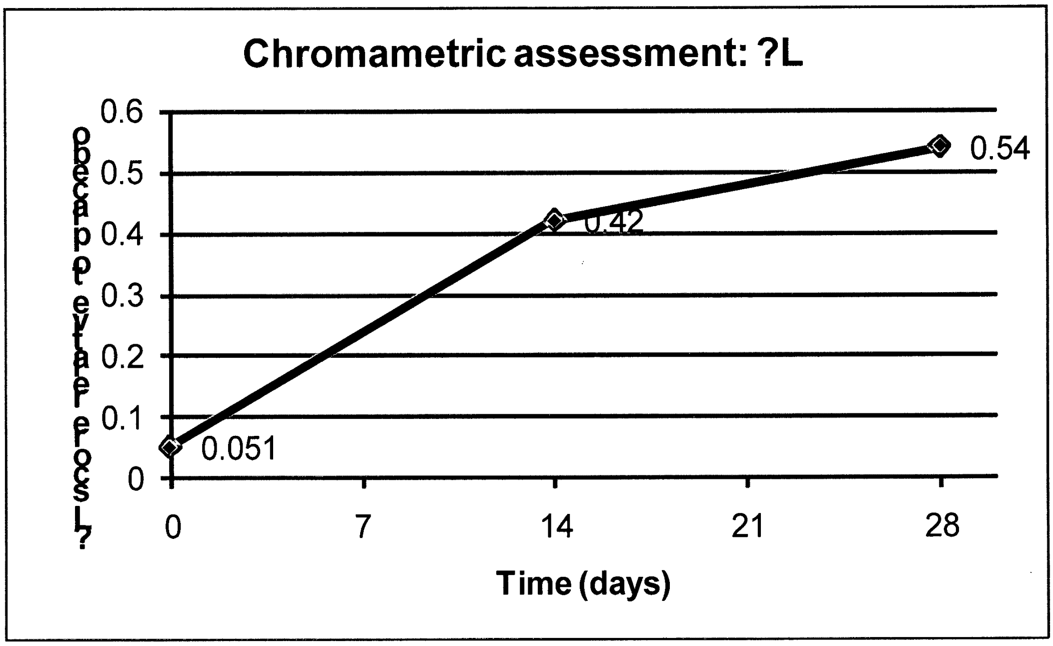


Fig. 3: The ΔL scores are averaged over the whole panel as a function of time. A positive value indicates that the arm treated with the active formulation was whiter than the arm with the placebo formulation, whereas the values at 0 week were not statistically significantly different from each other (p=0.363), this is the case at week 2 (p=0.0002) and week 4 (p=0.0001).

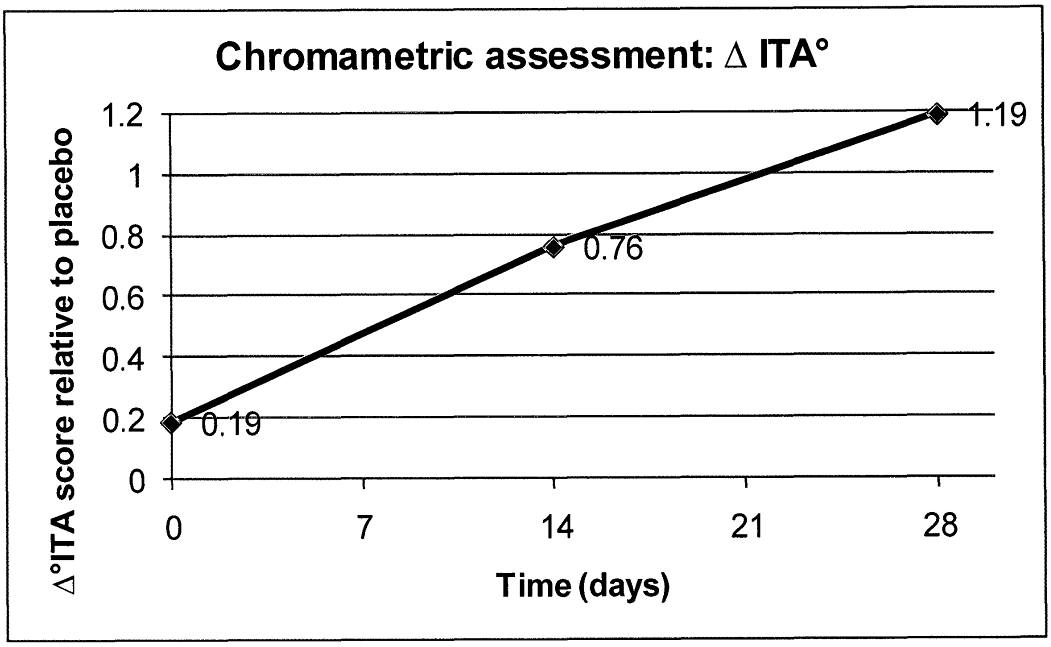


Fig.4: TheΔITA° scores are averaged over the whole panel as a function of time. A positive value indicates that the arm treated with the active formulation was whiter than the arm with the placebo formulation. Whereas the values at 0 week were not statistically significantly different from each other (p=0.21), this is the case at week 2 (p=0.003) and week 4 (p=0.001).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6596758 B1
- WO 9319743 A
- WO 0202074 A
- WO 9407462 A
- WO 9407837 A
- US 5753704 A
- EP 0662946 A
- WO 2006111233 A
- WO 2006111234 A
- DE 10244282
- DE 10232595
- US 6242099 B1
- WO 0009652 A
- WO 0072806 A
- WO 0071084 A
- EP 0671161 A
- DE 4116123 A
- DE 4308282 A

### Non-patent literature cited in the description


- **G. WEINDL et al.** *Drugs,* 2005, vol. 65 (14), 1919-1934
- **J. W. WIECHERS et al.** *International Journal of Cosmetic Science,* 2005, vol. 27, 123-132
- **HANLEY K et al.** *J Invest Dermatol,* April 1998, vol. 110 (4), 368-75
- **KOMUVES LG.** *J Invest Dermatol,* September 2000, vol. 115 (3), 353-60
- **L. MICHALIK ; W. WAHLI.** *Biochimica et Biophysica Acta,* 2007, vol. 1771, 991-998
- **Q. YANG et al.** *J. Invest. Dermatol.,* 2006, vol. 126, 374-385
- **TONTONOZ P ; HU E ; SPIEGELMAN BM.** *Cell,* 1994, vol. 79, 1147-1156
- **JO A. VAN GINDERACHTER et al.** *Blood,* 2006, vol. 108 (2), 525-535
- **SERTZING P. et al.** *American Journal of Clinical Dermatology,* 2008
- **LI Y ; QI Y ; HUANG TH ; YAMAHARA J ; ROUFOGALIS BD.** *Diabetes, obesity & metabolism,* 2008
- **KANG HY et al.** *Br J Dermatol.,* March 2004, vol. 150 (3), 462-8
- **WIECHERS, J. W.** *International Journal of Cosmetic Science.,* April 2005, vol. 27 (2), 123-132
- *Am J Physiol Endocrinol Metab,* 2007, vol. 293, E1159-E1168
- **HAUSMAN GJ et al.** *J. Animal Sci.,* 1982, vol. 54, 1286-1296
- *J. Animal Sci.,* 1982, vol. 54, 1286-1296
- **KERSHAW EE.** *J. Clin. Endocrinol. Metab.,* 2004, vol. 89-6, 2548-56
- **K. LEMANSKA ; H. SZYMUSIAK ; B. TYRAKOWSKA ; R. ZIELINSKI ; I.M.C.M. RIETJENS.** *Current Topics in Biophysics,* 2000, vol. 24 (2), 101-108
- **C.A. RICE-EVANS ; N.J. MILLER ; G. PAGANGA.** *Trends in Plant Science,* 1997, vol. 2 (4), 152-159
- **K. LEMANSKA ; H. SZYMUSIAK ; B. TYRAKOWSKA ; R. ZIELINSKI ; A.E.M.F. SOFFERS ; I.M.C.M. RIETJENS.** *Free Radical Biology&Medicine,* 2001, vol. 31 (7), 869-881
- *CHEMICAL ABSTRACTS,* 103 597-45-1
- *CHEMICAL ABSTRACTS,* 180 898-37-7
- *CHEMICAL ABSTRACTS,* 103 597-45-, 187 393-00-6
- **E.A. GALINSKI et al.** *Eur. J. Biochem.,* 1985, vol. 149, 135-139
- **STAELS, B et al.** *J. Clin. Invest.,* 1995, vol. 95, 705-712
- **FAJAS, L et al.** *J. Biol. Chem.,* 1997, vol. 272, 18779-18789
- **FORMAN, B. et al.** *Cell,* 1995, vol. 83, 803-812
- **SHER, T. et al.** *Biochemistry,* 1993, vol. 32, 5598-5604
- **SABIEN VAN NEERVEN et al.** *PPAR Research,* vol. 2007, 14
- **J. XU et al.** *Journal of Neuroimmunology,* 2006, vol. 176 (1-2), 95-105
- **MANGELSDORF, D.** *J.Cell,* 1995, vol. 83, 841-850
- **LI, M. et al.** *Development,* 2001, vol. 128, 675-688
- **CLIFFORD, J.** *EMBO J.,* 1996, vol. 15, 4142-4155

- **KULSKI et al.** *Journal of Molecular Evolution,* 2003, vol. 56 (4), 397-406
- **T. LENER et al.** *Experimental Gerontology,* 2006, vol. 41, 387-397
- **YASUFUMI MOROMIZATO et al.** *Am J Pathol.,* October 2000, vol. 157 (4), 1277-1281
- **HALLE LP et al.** *Arthritis Rheum,* 1989, vol. 32, 22-29
- **R. CHATELAIN et al.** *Archives of dermatological research,* October 1998, vol. 290 (9), 477-482
- **GARY J. FISHER et al.** *ARCH DERMATOL,* November 2002, vol. 138